# EUROPEAN PATENT SPECIFICATION

(11) **EP 2 729 000 B1**
(45) Date of publication and mention of the grant of the patent: **14.12.2022**
(21) Application number: 12807324.4
(22) Date of filing: 03.07.2012
(51) Int. Cl.: A01N 1/02

(54) **A METHOD FOR EXTENDED STORAGE OF RED BLOOD CELLS**
EINE METHODE ZUR VERLÄNGERTEN LAGERUNG ROTER BLUTKÖRPERCHEN
UN PROCÉDÉ POUR LE STOCKAGE PROLONGÉ DES ÉRYTHROCYTES

(30) Priority: 05.07.2011 US 201161504644 P; 05.07.2011 US 201161504640 P
(43) Date of publication of application: 14.05.2014
(73) Proprietor: Hemanext Inc., Lexington, MA 02421 (US)
(72) Inventor: YOSHIDA, Tatsuro, West Newton, Massachusetts 02465 (US); VERNUCCI, Paul, Billerica, Massachusetts 01862 (US)
(74) Representative: Bassil, Nicholas Charles
(86) International application number: PCT/US2012/045426
(87) International publication number: WO 2013/006631

(56) References cited:
- EP-B1- 1 109 447
- WO-A1-03/103390
- WO-A1-2011/046841
- WO-A1-2012/027582
- WO-A1-2012/061731
- US-A- 5 037 419
- US-A- 5 360 734
- US-A- 5 624 794
- US-A- 5 789 151
- US-A- 6 162 396
- US-A1- 2001 049 089
- US-A1- 2003 106 861
- US-A1- 2004 168 982
- US-A1- 2005 208 462
- US-A1- 2008 160 107
- US-B1- 6 358 678
- TATSURO YOSHIDA ET AL: "Anaerobic storage of red blood cells", BLOOD TRANSFUS, vol. 8, 1 January 2010 (2010-01-01), pages 220-36, XP055090172, DOI: 10.2450/2010.0022-10
- PAILLOUS N ET AL: "Mechanisms of photosensitized DNA cleavage", JOURNAL OF PHOTOCHEMISTRY AND PHOTOBIOLOGY B: BIOLOGY, ELSEVIER SCIENCE S.A., BASEL, CH, vol. 20, no. 2-3, 1 October 1993 (1993-10-01), pages 203-209, XP026728602, ISSN: 1011-1344, DOI: 10.1016/1011-1344(93)80152-Y [retrieved on 1993-10-01]
- PELLETIER ET AL: "Pathogen inactivation techniques", BEST PRACTICE & RESEARCH CLINICAL HAEMATOLOGY, ELSEVIER, AMSTERDAM, NL, vol. 19, no. 1, 1 March 2006 (2006-03-01), pages 205-242, XP005219070, ISSN: 1521-6926
- SEGHATCHIAN J ET AL: "Pathogen-reduction systems for blood components: The current position and future trends", TRANSFUSION AND APHERESIS SCIENCE, ELSEVIER SCIENCE, LONDON, GB, vol. 35, no. 3, 1 December 2006 (2006-12-01), pages 189-196, XP028058927, ISSN: 1473-0502, DOI: 10.1016/J.TRANSCI.2006.10.002 [retrieved on 2006-12-01]
- Boris Zavizion ET AL: "Inactivation of mycoplasma species in blood by INACTINE PEN110 process : INACTIVATION OF MYCOPLASMA SPECIES IN BLOOD", TRANSFUSION, vol. 44, no. 2, 1 February 2004 (2004-02-01), pages 286-293, XP055735683, US ISSN: 0041-1132, DOI: 10.1111/j.1537-2995.2004.00647.x
- HOLME ET AL: "Current issues related to the quality of stored RBCs", TRANSFUSION AND APHERESIS SCIENCE, ELSEVIER SCIENCE, LONDON, GB, vol. 33, no. 1, 1 August 2005 (2005-08-01) , pages 55-61, XP027724754, ISSN: 1473-0502 [retrieved on 2005-08-01]
- P ; Agarwal ET AL: "Effect of pre-storage gamma irradiation on red blood cells", Indian Journal of Medical Research Nov, vol. 122, no. 5, 1 January 2005 (2005-01-01), page 385, XP055464831,

## Description

### FIELD OF THE DISCLOSURE

The present disclosure relates to the systems and methods for the preservation of blood and red blood cells. More particularly, the disclosure relates to the systems and methods for the prolonged anaerobic storage of red blood cells from collection to transfusion.

### BACKGROUND OF THE INVENTION

The supplies of liquid blood are currently limited by storage systems used in conventional blood storage practice. Using current systems, stored blood expires after a period of about 42 days of refrigerated storage at a temperature above freezing (*i.e.*, 4°C) as packed blood cell preparations. Expired blood cannot be used and must be discarded because it will harm the ultimate recipient. One of the primary reasons for blood spoilage is its continued metabolic activity after it is stored. For example, in 2007, more than 45 million units of red blood cells (RBCs) were collected and stored globally (15.6 million in the US). During refrigerated storage, RBCs become progressively damaged by storage lesions. When transfused within the current 6-week limit, stored RBCs have lower quality (fraction of RBC removed; compromised O₂ delivery capacity) as well as potential toxicity, often manifested as side effects of transfusion therapy. These storage lesions are observed as altered biochemical and physical parameters associated with stored cells. Examples of these include *in vitro* measured parameters such as reduced metabolite levels (ATP and 2,3-DPG), reduced surface area, echinocytosis, phosphatidylserine exposure, and reduced deformability.

Stored blood undergoes steady deterioration which is partly caused by hemolysis, hemoglobin degradation and reduced adenosine triphosphate (ATP) concentration that occur during the storage period. These reasons and others limit the amount of readily available high quality blood needed for transfusions.

As discussed above, when RBCs are stored under refrigeration at temperatures above freezing (*e.g.*, 1-6°C, standard storage conditions) in a blood storage bag, away from mechanical stress and the constantly cycling environment of the circulation, the senescence process is partially suspended. However, with the lack of constant nutrient replenishment and waste removal under refrigerated storage, RBCs are gradually damaged, resulting in compromised physiological functions. By way of example, the following problems occur during extended storage:
- When RBCs are stored for an extended period, storage lesions accumulate and deteriorate RBCs and cause the up to 1% of RBCs to be hemolyzed during storage and up to 25% to be removed shortly after transfusion.
- Non-viable RBCs cause iron overload in chronically transfused patients.
- Transfusion does not always achieve the intended outcome of increased tissue perfusion.
- Hemoglobin in RBCs do not release oxygen efficiently at tissues due to loss of 2,3-DPG.
- RBCs are not able to enter and perfuse capillary beds due to loss of deformability.

Transfusing RBCs stored for longer periods may result in higher morbidity and longer hospital stays compared to transfusing "fresher" red cells. Higher morbidity and longer hospital stays result with RBCs that are stored longer than 6 weeks, in comparison to fresher red cells. For example, negative clinical outcomes in cardiac surgery occur when using 'older' blood; multiple organ failure in surgical patients reflecting the age of transfused red cells; correlation between older units and increased mortality in severe sepsis; failure to improve O₂ utilization attributed to decreased 2,3-DPG and decreased cardiac index associated with increased blood viscosity

This evidence suggests that the ineffectiveness and negative consequences of transfusion is attributable at least in part to the compromising effects of extended storage of RBCs. In addition to immediate removal by the recipient of certain RBCs, consequences of RBC storage lesions include: (i) Depletion of ATP (loss of RBC's ability to dilate the pre-capillary arteriole); (ii) Depletion of 2,3-DPG; (iii) Accumulation of oxidative damage caused by reactive oxygen species (ROS) formed by the reaction of denatured hemoglobin with O₂; and (iv) Decreased RBC deformability and increased RBC viscosity-caused in part by oxidative damage to membrane and cytoskeleton. Less deformable RBCs are excluded from capillary channels resulting in low capillary occupancy and reduced tissue perfusion. Massive transfusion of un-deformable cells may also contribute to multiple organ failure by blocking the organs' capillary beds. After transfusion, 2,3-DPG is synthesized relatively quickly *in vivo* to ~50% of the normal level in as little as 7 hours and to ~95% of the normal level in 2-3 days . However, since 2,3-DPG-depleted cells do not recover their levels immediately, O₂-carrying capacity is compromised to the detriment of critically ill patients requiring immediate O₂ delivery and tissue perfusion. There are numerous reports that emphasize the importance of RBCs with high oxygen carrying capacity in such clinical situations.

Storage of frozen blood is known in the art but such frozen blood has limitations. For a number of years, frozen blood has been used by blood banks and the military for certain high-demand and rare types of blood. However, frozen blood is difficult to handle. It must be thawed which makes it impractical for emergency situations. Once blood is thawed, it must be used within 48 hours. U.S. Patent No. 6,413,713 to Serebrennikov is directed to a method of storing blood at temperatures below 0°C.

U.S. Patent No. 4,769,318 to Hamasaki et al. and U.S. Patent No. 4,880,786 to Sasakawa et al. are directed to additive solutions for blood preservation and activation. U.S. Patent No. 5,624,794 to Bitensky et al., U.S. Patent No. 6,162,396 to Bitensky et al., and U.S. Patent No. 5,476,764 are directed to the storage of red blood cells under oxygen-depleted conditions. U.S. Patent No. 5,789,151 to Bitensky et al. is directed to blood storage additive solutions.

Additive solutions for blood preservation and activation are known in the art. For example, Rejuvesol (available from enCyte Corp., Braintree, MA) is added to blood after cold storage (*i.e*., 4°C) just prior to transfusion or prior to freezing (*i.e.*, at -80°C with glycerol) for extended storage. U.S. Patent No. 6,447,987 to Hess et al. is directed to additive solutions for the refrigerated storage of human red blood cells.

The effects of elevation and preservation of ATP levels in blood storage situations has been studied. For example, in "Studies In Red Blood Cell Preservation-7. In Vivo and in Vitro Studies With A Modified Phosphate-Ammonium Additive Solution," by Greenwalt et al., Vox Sang 65, 87-94 (1993), the authors determined that the experimental additive solution (EAS-2) containing in mM: 20 NH₄Cl, 30 Na₂HPO₄, 2 adenine, 110 dextrose, 55 mannitol, pH 7.15, is useful in extending the storage shelf-life of human RBCs from the current standard of 5-6 weeks to an improved standard of 8-9 weeks. Packed RBCs are suitable for transfusion following the removal of the supernatant with a single washing step. Greenwalt *et al.* also conclude that factors other than ATP concentration appear to play an increasingly important role in determining RBC viability after 50 days of storage. They cite the results of L. Wood and E. Beutler in "The Viability Of Human Blood Stored In Phosphate Adenine Media," Transfusion 7, 401-408 (1967), find in their own experiments that the relationship between ATP concentration and 24-hour RBC survival measurements appear to become less clear after about 8 weeks of storage. E. Beutler and C. West restate that the relationship between red cell ATP concentration and viability is a weak one after prolonged periods of storage in "Storage Of Red Cell Concentrates In CPD-A2 For 42 and 49 Days," J. Lab. Clin. Med. 102, 53-62 (1983).

In "Effects Of Oxygen On Red Cells During Liquid Storage at +4 °C.," by Hogman et al., Vox Sang 51, 27-34 (1986), the authors discuss that red cell content of ATP is slightly better maintained in anaerobic chamber than at ambient air storage after 2-3 weeks. Venous blood was refrigerated and deprived of additional oxygen during storage, by placing the oxygen-permeable storage bags in a nitrogen environment and thereby gradually reducing the level of oxygen saturation. The reduction in oxygen concentration occurs slowly during storage at 4 °C., and is far from complete, starting at about 60% and reaching about 30% hemoglobin saturation at 5 weeks. No conclusion could be drawn concerning the effects of this procedure on the overall quality of stored cells. These authors did not address or significantly reduce the oxygen-dependent damage to hemoglobin and the oxygen-mediated damage caused by hemoglobin breakdown products.

Many patents have addressed different aspects of blood storage. One such patent is U.S. Pat. No. 4,837,047 to Sato et al. which relates to a container for storing blood for a long period of time to keep the quality of the blood in good condition. Sato *et al.* is directed at improving the storage life of the stored blood by maintaining a partial pressure of carbon dioxide gas in the blood at a low level. Such partial pressure is apparently obtained through normalization with the outside atmosphere. The container is made of a synthetic resin film which has a high permeability to carbon dioxide gas for the purpose of making it possible for the carbon dioxide gas to easily diffuse from the blood to outside. However, the problems caused by the interaction of the oxygen and hemoglobin in the blood are not addressed.

Another patent, U.S. Pat. No. 5,529,821 to Ishikawa et al. relates to a container and a method for the storage of blood to prevent adhesion of the blood to the container. Blood is stored in containers composed of a sheet material having a plurality of layers where a first sheet which contacts the blood substantially prevents the activation and adhesion of blood platelets to the layer. Again, however, the problems caused by the interaction of the oxygen and hemoglobin in the blood are not addressed.

WO2012/061731 describes irradiation of red blood cells and anaerobic storage. WO2012/027582 describes a method for enhancing red blood cell quality and survival during storage. WO2011/046841 describes a blood storage bag system and depletion devices with oxygen and carbon dioxide depletion capabilities. Yoshida et al, Blood Transfus, (2010), vol. 8, pages 220 - 36, describes anaerobic storage of red blood cells. Pelletier et al (2006), Best Practice & Research Clinical Haematology, vol. 19, no. 1, pages 205-242 describes pathogen inactivation techniques. Seghatchian et al (2006) Transfusion And Apheresis Science vol. 35, no. 3, pages 189-196 describes the current position and future trends in pathogen-reduction systems for blood components.

In light of current technology, there is a need to improve the quality of red blood cells that are to be stored and to extend the storage life of such red blood cells in advance of transfusion to minimize morbidity associated with transfusions.

### SUMMARY OF THE INVENTION

To address such needs and others, the present disclosure includes and provides a system and methodology for the preservation of red blood cells is provided in which red blood cells are, e.g., oxygen and carbon dioxide depleted, undergo treatment and are stored in an anaerobic environment to optimize preparation for transfusion.

The invention is defined in the appended set of claims. The description may contain additional technical information, which although not part of the claimed invention, is provided to place the invention in a broader technical context and to illustrate possible related technical developments.

The invention provides a method for preparing red blood cells (RBCs) comprising:
separating RBCs from whole blood to form packed RBCs;
reducing oxygen and carbon dioxide from said packed RBCs to form oxygen and carbon dioxide-reduced packed RBCs;
inactivating pathogens in said packed RBCs with riboflavin and light therapy after said removing oxygen and carbon dioxide;
storing said oxygen and carbon dioxide-reduced packed RBCs in an anaerobic storage environment; and
irradiating said packed RBCs with gamma or x-ray irradiation, wherein said irradiation occurs before, during, or after said storing;
wherein said reducing oxygen and carbon dioxide from said packed RBCs comprises passing said packed RBCs through an oxygen and carbon dioxide depletion device,
wherein said irradiation before storing occurs after said reducing oxygen and carbon dioxide.

The method may further comprise adding an additive solution to said packed RBCs prior to said reducing oxygen and carbon dioxide from said packed RBCs to form a suspension of RBCs. Said additive solution may have a pH from 5.0 to 9.0. Said additive solution may further comprise an antioxidant selected from the group consisting of quercetin, alpha-tocopheral, ascorbic acid, and enzyme inhibitor for oxidase.

The method may further comprise one or more steps selected from the group consisting of:
adding nutrients or metabolic supplements to said oxygen and carbon dioxide-reduced packed RBCs, or oxygen and carbon dioxide-reduced suspension of RBCs during storage in said anaerobic storage environment, and
reoxygenating the stored oxygen and carbon dioxide-reduced packed RBCs, or oxygen and carbon dioxide-reduced suspension of RBCs to form oxygenated packed RBCs or an oxygenated suspension of RBCs for transfusion.

Said oxygen and carbon dioxide depletion device may comprise:
a housing,
a plurality of structures selected from the group consisting of hollow fibers, gas-permeable films, and gas permeable fibers, and any combinations thereof extending within the housing from an entrance to an exit thereof; wherein said plurality of structures are formed of a material that is permeable to both oxygen and carbon dioxide and are adapted to receiving and conveying RBCs; and
an amount of an oxygen sorbent and carbon dioxide sorbent packed within the housing and contiguous to said plurality of structures.

Said anaerobic storage environment may further comprise a carbon dioxide depleted storage environment containing oxygen sorbents and carbon dioxide sorbents for maintaining the oxygen and carbon dioxide-reduced packed RBCs or oxygen and carbon dioxide-reduced suspension of RBCs in an oxygen and carbon dioxide depleted storage environment.

Said anaerobic storage environment may comprise an oxygen-impermeable storage bag containing an oxygen and carbon dioxide sorbent to maintain said oxygen and carbon dioxide-reduced packed RBCs, or oxygen and carbon dioxide- reduced packed RBCs in an oxygen and carbon dioxide reduced condition.

The method may further comprise leukoreducing said whole blood, said packed RBCs, or said suspension of RBCs, wherein said leukoreduction occurs prior to or after reducing oxygen and carbon dioxide, and before, during or after said storing. The method may further comprise one or more steps selected from the group consisting of:
editing a composition selected from the group consisting of said whole blood before said storing, said packed RBCs before said storing, said suspension of RBCs before said storing, the stored oxygen and carbon dioxide-reduced packed RBCs, and the stored oxygen and carbon dioxide- reduced suspension of RBCs,
adding nitric oxide or NO precursor molecules to said oxygen and carbon dioxide reduced packed RBCs, or oxygen and carbon dioxide- reduced suspension of RBCs during or after said storing, and
reducing the volume of said oxygen and carbon-dioxide reduced packed RBCs, or oxygen and carbon dioxide- reduced suspension of RBCs after said storing.

Said editing may comprise identifying and removing moribund RBCs,
wherein said identifying moribund RBCs is selected from the group consisting of:
   removing the densest 10% of RBCs by filtration;
   osmotically shocking RBCs and removing damaged RBCs;
   removing RBCs by high affinity ligand filtration; and
   separating RBC with reduced deformability by processing through a bump array microfluidic device;
wherein said editing occurs before said separating RBCs from whole blood, after said separating RBCs from whole blood, before said storing, or after said storing.

Said high affinity ligand filtration may comprise binding of said RBC using a high affinity ligand that recognizes clustered Band-3 or phosphatidylserine on the surface of said RBC.

The method may further comprise:
editing a composition selected from the group consisting of said whole blood before said storing, said packed RBCs before said storing, and the stored oxygen and carbon dioxide-reduced RBCs;
reducing the volume of said stored oxygen and carbon dioxide-reduced packed RBCs prior to reoxygenation; and
adding nitric oxide to said stored oxygen and carbon dioxide-reduced packed RBCs when said oxygen and carbon dioxide-reduced packed RBCs are stored in said anaerobic storage environment.

Said irradiating said packed RBCs with gamma or x-ray irradiation occurs after said reducing oxygen and carbon dioxide and before said storing. Said irradiating said packed RBCs with gamma or x-ray irradiation may occur during said storing.

The present disclosure includes a method for extended storage of red blood cells from collection to transfusion that optimizes red blood cells prior to transfusion.

The present disclosure provides for, and includes, a method for preparing red blood cells (RBCs) including obtaining whole blood, separating the RBCs from the whole blood to form packed RBCs, depleting oxygen to form oxygen depleted RBCs or depleting oxygen and carbon dioxide to form oxygen and carbon dioxide depleted RBCs and storing the oxygen depleted or oxygen and carbon dioxide depleted RBCs in an anaerobic storage environment to maintain an oxygen depleted or oxygen and carbon dioxide depleted condition.

In aspects of the present disclosure, the method may further include adding an additive solution to the packed RBCs to form a suspension. In some aspects the additive solution may include AS-1, AS-3, AS-5, SAGM, PAGG-SM, PAGG-GM, MAP, SOLX, ESOL, EAS61, OFAS1 or OFAS3 alone or in combination. In a further aspect, the additive solution may have a pH from 5.0 to 9.0. In another aspect, the additive may include an antioxidant. In some aspects according the present disclosure, the antioxidant may be quercetin, alpha-tocopheral, ascorbic acid, or enzyme inhibitors for oxidases.

Not forming part of the invention is an integrated blood storage system and method that can include an oxygen and carbon dioxide removal system, a blood storage system and a pre-transfusion procedure to prepare the stored blood for transfusion.

Additionally, the present disclosure also includes a method that may incorporate leukoreduction and editing steps to optimize RBCs in preparation for transfusion. Leukoreduction may include removing white blood cells that can carry viruses and cause fevers. Editing can include removing RBCs that exhibit indications of being compromised.

Accordingly, the present disclosure also provides a novel procedure for blood storage which addresses at least the problems of hemoglobin degradation, red blood cell lysis (hemolysis) and ATP and 2-3 DPG depletion in a manner consistent with the practice of autologous transfusion and enhanced heterologous transfusion logistics, and which achieves significant prolongation of the time during which refrigerated storage of red blood cells is not detrimental to their subsequent use.

The present disclosure further provides for a methodology for enhancing the effect of irradiation and stabilizing red cells prior to storage or during/after storage in preparation for transfusion.

The present disclosure further provides for a methodology for reducing the growth of aerobic bacteria and parasites present in red blood cells prior to storage or during storage in preparation for transfusion.

The present disclosure further provides for a methodology for minimizing hemolysis and morphology changes of red blood cells during storage in non DEHP storage bags.

The present disclosure further provides for a methodology for stabilizing and enhancing pathogen inactivation of red blood cells prior to storage or during storage in preparation for transfusion.

The present disclosure, still further provides for a methodology for providing nitric oxide to red blood cells during storage, after storage and immediately prior to transfusion, e.g., to permit vasodilation of the vessels of the recipient of the RBCs.

The present disclosure, still yet further provides for a methodology for reducing the volume of red blood cells after storage and re-oxygenating such RBCs immediately prior to transfusion.

### BRIEF DESCRIPTION OF THE DRAWINGS

FIG. 1 illustrates an exemplary flowchart of the components and methodology from blood collection to transfusion using a blood anaerobic storage system of the present disclosure;
FIG. 2 illustrates an exemplary system according to the FIG. 1 of the present disclosure in which, blood is collected, components are separated, optional additive solution is added to packed RBC, leukoreduced then stored anaerobically;
FIGS. 3a and 3b illustrate the effects of, oxygen and oxygen and carbon dioxide depletion on ATP and DPG, respectively, during extended storage in OFAS3 additive solution;
FIGS. 4a and 4b illustrate a partial detailed perspective view of an RBC inlet portion of the combination leukoreduction filter and O₂/CO₂ depletion device according to the system of Fig. 5;
FIG. 5 illustrates a pre-storage oxygen, carbon dioxide oxygen and carbon dioxide depletion device of the present disclosure;
FIG. 6 illustrates a cross-section view of the depletion device of the device of FIG. 5;
FIGS. 7A through 7D illustrate cross-section views of depletion devices;
FIG. 8 illustrates the starting and ending partial pressures of oxygen and carbon dioxide, respectively, in red blood cells;
FIG. 9 illustrates the ending partial pressure of oxygen in RBCs as a function of flow rate of RBCs and how depletion varies according to flow rate of RBCs when device similar to Fig 5 was fed with RBC suspension of 16.5 Torr;
FIG. 10 illustrates alternative oxygen/carbon dioxide depletion device incorporating leukoreduction and plasma separation components;
FIGS. 11a and 11b illustrate alternative blood bags according to the present disclosure;
FIGS. 12A through 12B illustrate blood storage bags according to the present disclosure;
FIG. 13 illustrates an alternative configuration according to the present disclosure;
FIG. 14 illustrates an aspect for volume reduction;
FIG. 15 illustrates a comparison of ATP of RBCs stored in accordance with the present disclosure;
FIG. 16 illustrates a comparison of 2,3 DPG of RBCs stored in accordance with the present disclosure;
FIG. 17 illustrates a comparison of hemolysis of RBCs stored in accordance with the present disclosure;
FIG. 18 illustrates a transfusion kit having an oxygenation device to oxygenate RBCs in advance of transfusion;
FIG. 19 illustrates an alternative configuration according to the present disclosure, including leukoreduction, oxygen, carbon dioxide or oxygen and/or carbon dioxide depletion;
FIG. 20 illustrates an alternative configuration according to the present disclosure, including leukoreduction, oxygen, carbon dioxide or oxygen and/or carbon dioxide depletion and irradiation at different times during aerobic and anaerobic conditions of RBCs;
FIG. 21 illustrates an alternative configuration according to the present disclosure, including leukoreduction, oxygen, carbon dioxide or oxygen and/or carbon dioxide depletion and re-oxygenation immediately prior to transfusion to a recipient;
FIG. 22 illustrates an alternative configuration according to the present disclosure, including leukoreduction, oxygen, carbon dioxide or oxygen and/or carbon dioxide depletion, and pathogen inactivation at various possible times during collection and storage; and
FIG. 23 illustrates an alternative configuration according to the present disclosure, including leukoreduction, oxygen, carbon dioxide or oxygen and/or carbon dioxide depletion, and nitric oxide addition at various possible times during storage.

### DETAILED DESCRIPTION

The transfusion of red blood cells (RBCs) is a life-saving therapy aimed at improving oxygenation of the tissues and vital end organs in severely anemic patients. The majority of RBC units used for transfusion are stored at 1-6°C for up to 42 days in an oxygen-permeable polyvinylchloride blood bag that contains additive / preservative solution.

### Exemplary Definitions:

Blood Donor: Whole blood is preferably donated from a healthy individual or donor and held in a blood bank for later use to be ultimately used by a recipient. Subjects who are scheduled for surgery or other treatment may donate blood for themselves in a process known as autologous blood donation. Alternatively, blood is donated for use by another in a process known as heterologous transfusion. The collection of a whole blood sample drawn from a donor, or in the case of an autologous transfusion from a patient, may be accomplished by techniques known in the art, such as through donation or apheresis.

Whole Blood: Whole blood is a suspension of blood cells that contains red blood cells, white blood cells, platelets suspended in plasma, including electrolytes, hormones, vitamins, antibodies, etc.

Red Blood Cells (RBCs): Human red blood cells *in vivo* are in a dynamic state. In whole blood, white blood cells are normally present in the range between 4,300 and 10,800 cells/µL and the normal RBC range at sea level is 5.4 million/µL (+ 0.8) for men and 4.8 million µL (+ 0.6) for women. The red blood cells contain hemoglobin, the iron-containing protein that carries oxygen throughout the body and gives red blood its color. The percentage of blood volume composed of red blood cells is called the hematocrit. Packed red blood cells may be prepared from whole blood using centrifugation techniques commonly known in the art. In an aspect according to the present disclosure, the packed red blood cells may be the blood component that is stored in the storage system for later transfusion.

The normal life span of a red blood cell (RBC) is 120 days. Approximately 0.875% of the RBCs are retired every 24 hours by the spleen and new RBCs are made by the bone marrow. Consequently, when blood is drawn from a donor, there are a percentage of white blood cells and a spectrum of cells of different ages.

The main function of RBCs is to exchange oxygen and carbon dioxide at lung and tissues, and unlike other cells in body, it does not rely on oxygen in oxidative phosphorylation but entirely on glycolysis for ATP production. ATP is critical for viability of RBC and together with 2,3-diphosphoglycerate (2,3-DPG), their free cytosolic concentrations are tightly regulated by their function on feedback inhibition to key enzymes in glycolytic pathway. Under a refrigerated storage condition, dis-inhibition of glycolytic pathway is desirable to overcome the gradual depletion of ATP and 2,3-DPG over several weeks of storage. Hemoglobin concentration in RBC is similar to 2,3-DPG and ATP, and its deoxygenated state has a binding pocked with high affinities for 2,3-DPG and ATP compared to oxy-hemoglobin. Thus, stripping this oxygen to few % occupancy (~60% occupied when collected and processed) will cause uptake of 2,3-DPG and ATP, resulting in reduced concentration of free molecules, stimulating glycolytic flux.

Platelets: The platelets are small cellular components of blood that facilitate the clotting process by sticking to the lining of the blood vessels. The platelets like the red blood cells are made by the bone marrow and survive in the circulatory system for 9 to 10 days before they are removed by the spleen. Platelets are typically prepared using a centrifuge to separate the platelets from the plasma.

Plasma: Plasma is a protein-salt solution and the liquid portion of the blood in which red and white blood cells and platelets are suspended. Plasma is 90% water and constitutes about 55 percent of the blood volume. One of the primary functions of plasma is to assist in blood clotting and immunity. Plasma is obtained by separating the liquid portion of the blood from the cells. Typically, plasma is separated from the cells by centrifugation. Centrifugation is the process used to separate the components of the whole blood into the plasma, the white blood cells, the platelets and the packed red blood cells. During centrifugation, the plasma will initially migrate to the top of a vessel during a light spin. The plasma is then removed from the vessel. The white blood cells and platelets are removed during a second centrifugation cycle to produce the packed red blood cells. This application will discuss an efficient alternative to using a centrifuge that minimizes the cost of traditionally used instrumentation.

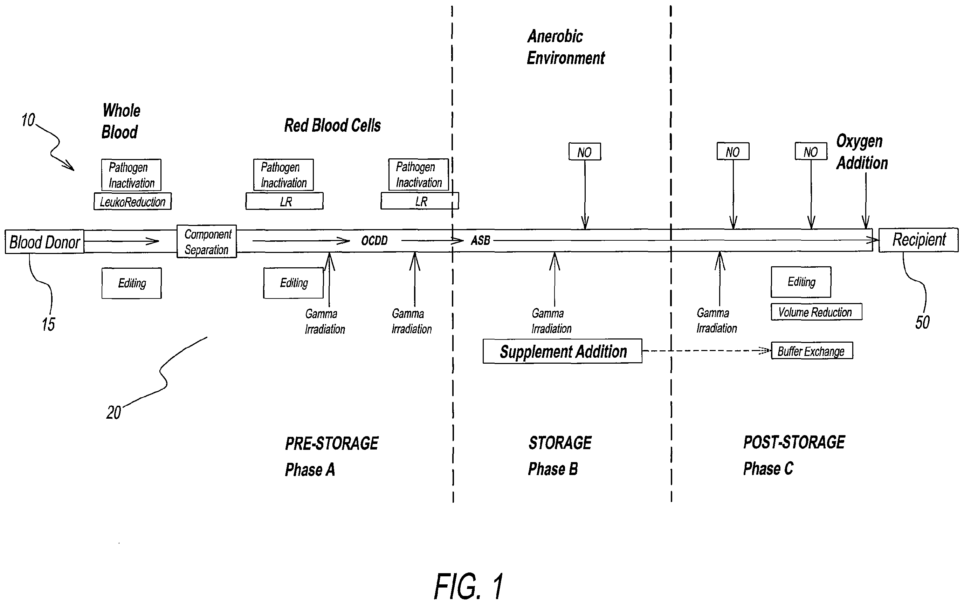

In its most general form, the present disclosure provides for, and includes, an integrated system and method for the preparation and extended storage of red blood cells, from receipt of whole blood from a donor until transfusion to a recipient. However, the invention is defined in the claims. By way of example, FIG. 1 illustrates an exemplary flowchart of the components and methodology from blood collection from a blood donor 15 to transfusion to a recipient 50 using a anaerobic storage method 10 and system 20 through Pre-Storage Phase A, Storage Phase B in an anaerobic environment, and Post-Storage Phase C.

By way of illustration, method 10 describes a storage system 20 that includes an optional additive addition, and oxygen, carbon dioxide, or oxygen and carbon dioxide (collectively referred to herein as O/CD) depletion of RBCs before and during storage, together with enhancing treatments include leukoreduction, editing, pathogen reduction, irradiation and nitric oxide treatment and oxygen addition to enhance the quality of stored RBCs and to optimize the transfusion process to a recipient and reduce morbidity associated with such transfusion.

Again referring to the drawings, and particular to FIG. 1, a method 10 describes storage system 20 from collection from a donor 15 to transfusion to a recipient 50. System 20 shows a method that has three phases during which different sub-processes or steps may occur. The three phases are generally: Pre-Storage Phase A, Storage Phase B and Post-Storage Phase C. As shown in FIG. 1, different steps of the blood storage process 20 can occur at different phases to achieve optimal blood transfusion results. For example, but not forming part of the invention, irradiation can optionally occur during Pre-Storage Phase A before oxygen removal, during Storage Phase B, during the Post-Storage Phase C, during Storage Phase B and a portion of Pre-Storage Phase A and Post-Storage Phase C, or combinations thereof, etc. Similarly, editing of RBCs (*e.g.*, to remove moribund RBCs) can occur during Pre-storage Phase A, during Post-storage Phase C, or a combination thereof, etc. The anaerobic environment has synergistic relationships with steps such as the addition of nitric oxide, irradiation and pathogen inactivation, that provide advantages to the RBCs that must occur in such anaerobic environment, as will be discussed below. Accordingly, there exist several different alternative sequences, not forming part of the invention, for the blood storage processing according to the present disclosure.

Pre-storage Phase A, includes the time from collection from a donor to storage in an anaerobic environment. During Phase A, whole blood may be collected from a donor, and the blood components, namely, plasma, platelets and RBCs may be separated. An optional additive solution may be added to the whole blood to aid in storage and/or processing, as further described herein. Processing such as leukoreduction and editing may occur during Pre-storage Phase A. During Phase A, oxygen and carbon dioxide (O/CD) are depleted prior to Storage Phase B. O/CD may be depleted either by an oxygen, or oxygen and carbon dioxide depletion device (OCDD).

Storage Phase B is an anaerobic storage period, wherein RBCs are stored in an anaerobic storage environment.

Post-Storage Phase C, after storage in an anaerobic storage environment but prior to transfusion to recipient. Post-Storage Phase C may include processing such as volume reduction, editing, cleansing during buffer exchange, the addition of either or both nitric oxide and oxygen, etc.

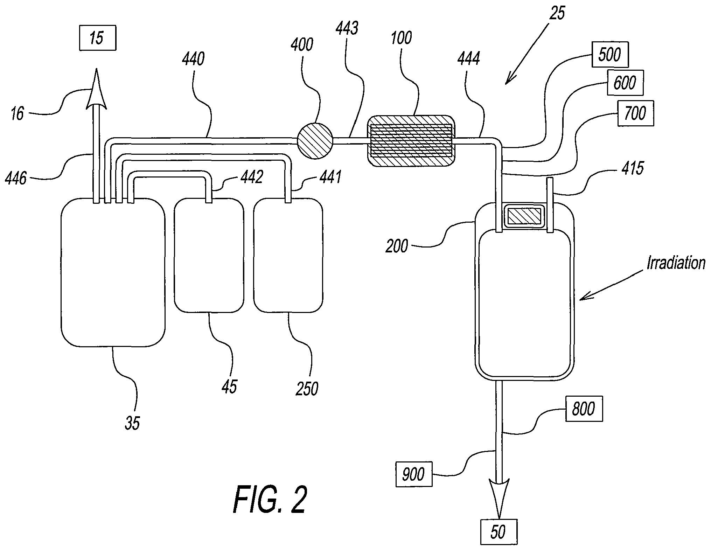

Referring to the drawings and in particular to FIG. 2, an exemplary anaerobic storage system, which does not form part of the invention, is shown and referenced using reference numeral 25. System 25 may be constructed so as to be disposable. Again, system 25 is an exemplary system, accordingly, different sub-processes or steps can occur at different times or during different phases as discussed above. Blood storage system 25 includes an oxygen/carbon dioxide depletion device 100 (OCDD 100), an anaerobic blood storage bag 200 and an optional additive solution bag 250. Components conventionally associated with the process of blood collection are a phlebotomy needle 16, a blood collection bag 35 containing an anti-coagulant and a bag 45 containing plasma. Tubing can connect the various components of the blood storage system 25 in various configurations (one instance shown). OCDD 100 removes oxygen and carbon dioxide from red blood cells traveling therethrough. System 25 may also contains a leukoreduction filter 400, and editing device 500, an irradiation device 600, a pathogen inactivation device 700, a volume reduction device 800 and a nitric oxide device 900 to immediately supply nitric oxide to the RBCs in advance of transfusion to a recipient 50. System 25 can contain all or a combination of such devices 400 through 900 in varying configurations as discussed below.

Components of system 25 are connected in a convention fashion. Tube 440 connects collection bag 35 with leukoreduction filter 400. Tube 441 connects solution bag 250 with collection bag 35. Tube 442 connects plasma bag 45 with collection bag 35. Tube 443 connects leukoreduction filter 400 with OCDD 100. Tube 444 connects OCDD 100 with blood storage bag 200. Blood storage system 25 is preferably a single-use, disposable, low cost system.

System components, namely, leukoreduction filter 400, editing device 500, irradiation device 600, pathogen inactivation device 700, volume reduction device 800 and nitric oxide device 900, perform various therapies for the RBCs prior to transfusion. Depending upon the therapies, such therapies are preferably performed on RBCs before passage through OCDD or after storage in storage bag 200. After being depleted in O/CD, RBCs are maintained in an oxygen, carbon dioxide, or oxygen and carbon dioxide depleted environment to ensure the desired results for the patient and to avoid morbidity commonly associated with transfusions using stored RBCs.

In certain aspects, if desired, after packed RBCs are collected from whole blood obtained from donor 15, an optional additive solution, e.g., from bag 250 may be provided to the packed RBCs to form a suspension of packed RBCs. Additive solutions may generally help to prevent rapid deterioration of RBCs. Additive solution bag 250 may include an additive solution optimized for anaerobic storage. For each of the several instances addressed herein, an additive solution from bag 250 may be provided prior to depleting O/CD from the RBCs. By way of example, between 50-300 ml of additive solution/unit of packed RBCs (450 - 500 ml whole blood draw) may be added. In certain aspects, 100 to 110 ml of additive solution per unit of packed RBCs may be added. In another aspect, 50 to 100 ml of additive solution per unit of packed RBCs may be added. In an aspect according the present disclosure, the 75 to 125 ml of additive solution per unit of packed RBCs may be added. In yet another aspect according the present disclosure, the 90 to 120 ml of additive solution per unit of packed RBCs may be added.

By way of example, the additive solution may include an aqueous solution of adenine, dextrose, mannitol, citrate ion, and dihydrogen phosphate ion. Alternatively, additive solutions may include AS-1, AS-3, AS-5, SAGM, PAGG-SM, PAGG-GM, EAS61, OFAS1, OFAS3, MAP, ESOL, SOLX and any combinations thereof. (See, Rossi's Principles of Transfusion Medicine 4th edition, Simon,T; Snyder, E, et al. Wiley-Blackwell; M Shimizu, H Fujii, H Mizoguchi, M Masuda, K Toyama, Rinsho Ketsueki et al., "Multicenter clinical evaluation of red cell concentrates stored up to 6 weeks in MAP, a new additive solution," The Japanese Journal 33:148 (1992); Dumont LJ, Yoshida T, AuBuchon JP, "Anaerobic storage of red blood cells in a novel additive solution improves in vivo recovery," Transfusion 49:458-64 (2009); U.S. Patent No. 5,789,151, issued August 4, 1998, entitled "Prolonged cold storage of red blood cells by oxygen removal and additive usage," issued August 4, 1998; U.S. Patent No. 4,769,318 issued September 6, 1988 to Hamasaki et al. entitled "Additive Solution for Blood Preservation and Activation"; and U.S. Patent No. 6,162,396 to Bitensky et al. issued December 19, 2000 entitled "Blood Storage Device and Method for Oxygen Removal").

Additive solution OFAS3 includes adenine, dextrose, mannitol, NaH₂PO₄, and optionally NaCl and/or NH₄Cl. Additive solution OFAS3 preferably includes ingredients having the following ranges: about 0.5-4.0 mmole/liter of adenine, about 50-150 mmole/liter of dextrose, about 20-70 mmole/liter of mannitol, about 0-100 mmole/liter of NaCl, about 2-20 mmole/liter of NaH₂PO₄, and about 0-30 mmole/liter NH₄Cl. Preferably OFAS3 has an adjusted pH from about 5.5-7.5 and includes about 2 mmole/liter adenine, about 110 mmole/liter dextrose, about 55 mmole/liter NaCl, and about 12 mmole/liter NaH₂PO₄ and an adjusted pH of about 6.5. Additional instances of OFAS3 are provided in U.S. Patent No. 8,071,282, issued December 6, 2011.

**Table 1**

| Ingredient | Range (mM) |
|---|---|
| Adenine | 0.5-4.0 |
| Dextrose | 50-150 |
| Mannitol | 0-70 |
| NaCl | 0-100 |
| NaH₂PO₄ | 2-20 |
| NH₄Cl | 0-30 |
| | |
| Effective Osm | 100 - 300 |
| Adjusted pH | 5.0 - 7.7 |
| mL added | 100-300 |

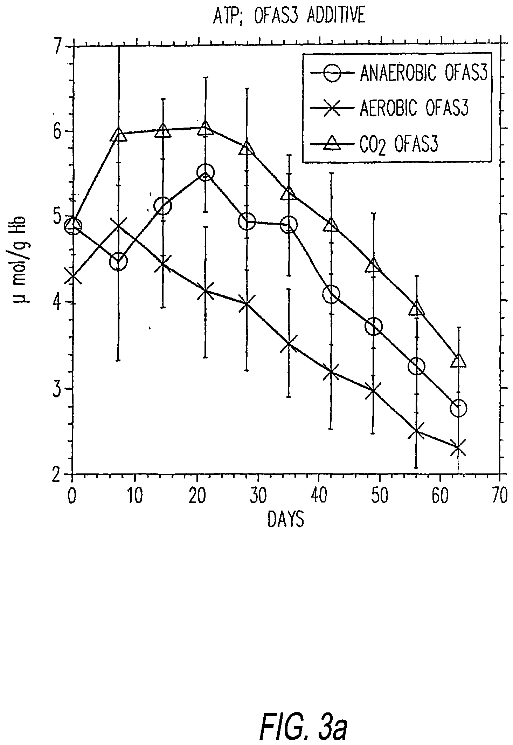

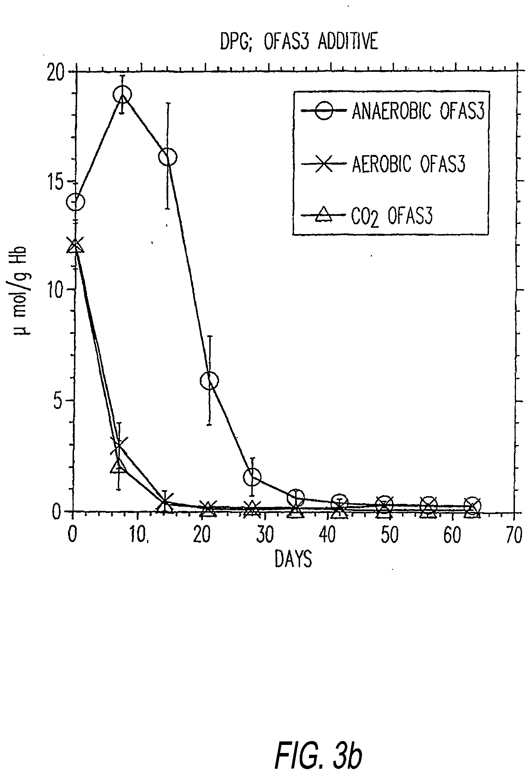

OFAS3 has shown enhanced ATP levels and good *in vivo* recovery as disclosed herein. FIG. 3a shows the effects of oxygen and oxygen and carbon dioxide depletion on ATP during extended storage in oxygen depleted or anaerobic OFAS3 additive solution. FIG. 3b shows the effects oxygen and oxygen and carbon dioxide depletion on 2, 3 DPG during extended storage in oxygen depleted or anaerobic OFAS3 additive solution. The highest ranges are from 8 to 30 days for ATP and from 0 to 20 days for 2, 3 DPG. Ideally, RBCs would be transfused to recipient 50 during such length of time.

To increase the time of acceptable *in vivo* recovery of RBCs in liquid storage, attempts have been made to improve additive solutions and storage processes. In "Studies In Red Blood Cell Preservation-7. In vivo and in vitro Studies With A Modified Phosphate-Ammonium Additive Solution," by Greenwalt et al., Vox. Sang. 65:87-94 (1993), the authors determined that the experimental additive solution (EAS-2) containing in mM: 20 NH₄Cl, 30 Na₂HPO₄, 2 adenine, 110 dextrose, 55 mannitol, pH 7.15, is useful in extending the storage shelf-life of human RBCs from the current standard of 5-6 weeks to an improved standard of 8-9 weeks. However, packed RBCs stored in the medium were not directly infusible but required the removal of the supernatant with a washing step prior to transfusion due to the presence of ammonium in the additive solution.

In other instances, the additive solution may include antioxidants. Particularly preferred are antioxidants that may be active under minimal oxygen conditions and therefore, potentially synergistic in their action in an anaerobic storage environment. For instance, the antioxidant may be selected from quercetin and other bioflavonoids, alpha-tocopheral, ascorbic acid, ebselen, oxypurinol, hydrocortisone and other enzyme (oxidase) inhibitor molecules,and combinations thereof. Quercetin, a flavonoid with antioxidant activity is safe and efficient to act as an antioxidant when administered clinically. Quercetin scavenges oxygen radicals, inhibits lipid peroxidation *in vitro,* and has been shown to reduce erythrocyte membrane damage. In certain instances, the antioxidant may be a flavonol. In other aspects, the flavonoid may be rutin or epicatechin. Ascorbic acid is very effective anti-oxidant, but can also function as pro-oxidant. However, since relatively high concentrations (~10 mM, concentration necessary to be effective in stored blood) and presence of iron (free or heme) and oxygen are necessary for its pro-oxidant activity, the anaerobic conditions of the present disclosure should provide a low effective concentration of use without concern for an pro-oxidant activity.

### Leukoreduction

As shown in FIG. 1, the whole blood, packed RBCs, or suspension of RBCs may undergo leukoreduction 400. Leukoreduction is the general process of removing white blood cells from whole blood or red blood cells. As shown, the leukoreduction may occur prior to or after depleting oxygen, carbon dioxide or oxygen and carbon dioxide (O/CD) to form O/CD depleted RBCs, and before, during or after storing in said anaerobic storage environment.

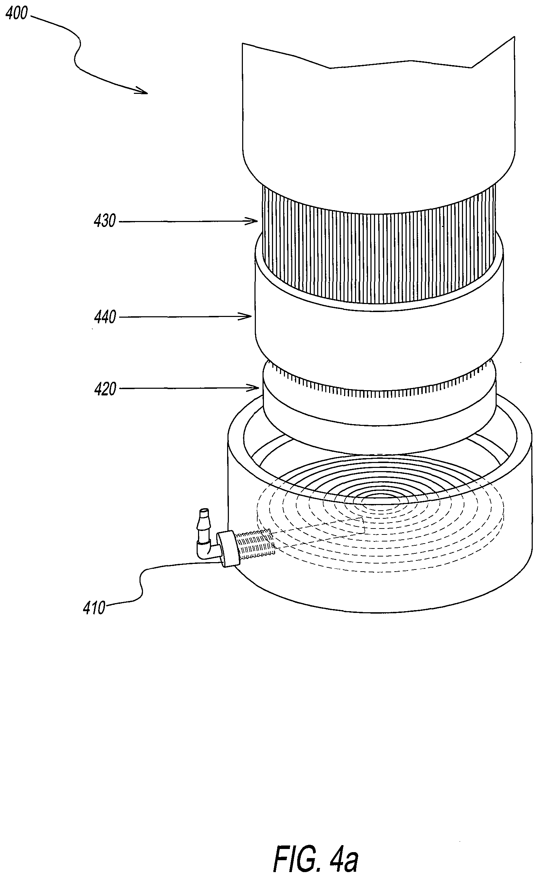

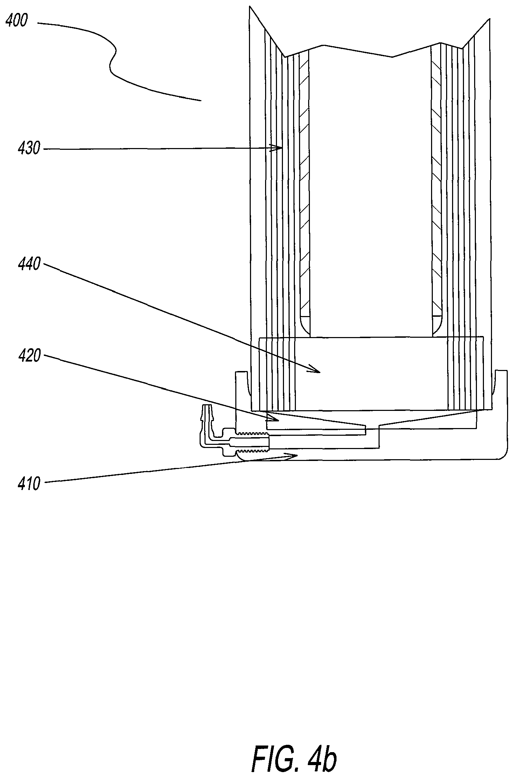

In accordance with certain aspects, referring to FIGS. 4a and 4b, a combination leukoreduction filter and OCDD 400 is shown. Combination leukoreduction and OCDD filter 400 includes an inlet flow distributor 410, a leukoreduction media 420, a plurality of hollow fibers and/or gas-permeable films or fibers 430, and a fiber/film support 440 to hold the plurality of fibers and/or gas-permeable films or fibers 430. Plurality of hollow fibers and/or gas-permeable films or fibers 430 are for the purpose of removing oxygen and or carbon dioxide from red blood cells and will be discussed further below in conjunction with OCDD 101.

Leukoreduction media 420 is preferably a fibrous or a felt-like filtering material that captures leukocytes (e.g., Pall Corporation), prior to such leukocytes travelling through plurality of hollow fibers and/or gas-permeable films or fibers 430 for oxygen, carbon dioxide, or oxygen and carbon dioxide depletion. In some aspects according to the present disclosure, the leukoreduction media 420 may be a LeukoGuard-6^{®} type filter media. In an aspect the leukoreduction media 420 may be Leukotrap^{®} Affinity Plus Prion and Leukocyte Reduction Filter media. In an aspect the leukoreduction media 420 may be Leukotrap^{®} media. Leukoreduction media 420 may comprise a fibrous medium, for example a medium prepared from melt-blown fibers, as disclosed in, for example, U.S. Pat. Nos. 4,880,548; 4,925,572, 5,152,905, 5,443,743, 6,231,770, and 7,361,277. Each of the media, which can be preformed media, can include a plurality of layers, as disclosed in the U.S. Patents listed above. Fiber/film support 440 supports the plurality of fibers/films 430 in a vertical configuration and may be made from a material such as polyurethane or a similar material. Either whole blood or RBCs flow through media 420 leukoreduction process.

Method 10 shows that leukoreduction filter 400 or the process of leukoreducing can optionally occur at whole blood stage or after RBCs have been separated from the plasma and platelets, before oxygen and carbon dioxide have been removed or after oxygen and/or carbon dioxide depletion in OCDD. In either case, leukoreduction can occur before storage of RBCs in a blood storage bag 200.

The benefits of leukoreduction are several. Leukoreduction may reduce the likelihood of fever in recipients, enhance RBC storage characteristics and reduce the transmission of viruses contained in leukocytes. Leukocytes in blood products can cause immunosuppressive effects and can pre-dispose recipients to an increased risk of infections, and may serve as vehicles of pathogen transmissions. Leukoreduction may reduce RBC storage lesions, reduce primary alloimunization and reduce the total number of transfusion reactions in a recipient. By removing leukocytes from the RBCs before storage in storage bag 200, the deleterious effects of leukocytes highlighted above can be avoided and the quality of stored RBCs may be thereby increased or enhanced.

### Oxygen/Carbon Dioxide Removal

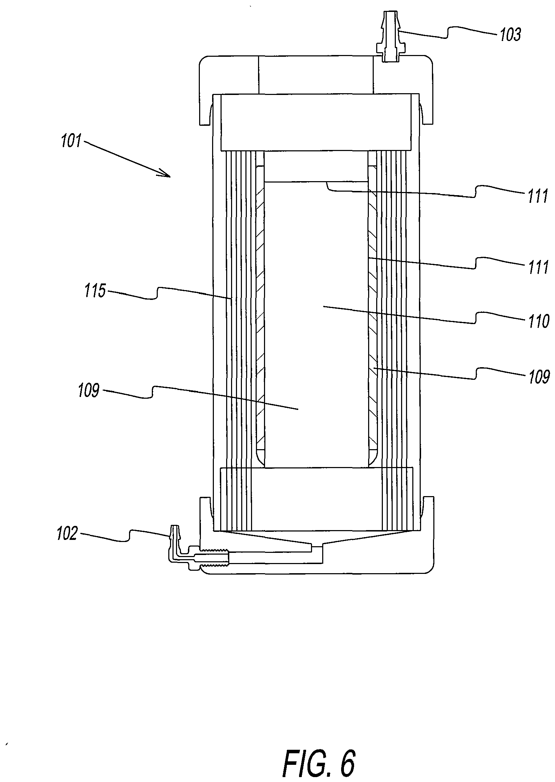

The presently claimed method comprises reducing oxygen and carbon dioxide from packed RBCs to form oxygen and carbon dioxide-reduced packed RBCs, wherein reducing oxygen and carbon dioxide from packed RBCs comprises passing packed RBCs through an oxygen and carbon dioxide depletion device. As also disclosed herein, RBCs may be treated to remove oxygen, carbon dioxide or oxygen and carbon dioxide in OCDD 101, as shown in FIGS. 2, 5 and 6. OCDD 101 may have a housing 104, an inlet port 102 and an exit port 103, through which RBCs enter and exit OCDD 101, respectively. OCDD 101 of FIG. 6 represents one OCDD and contains an oxygen sorbent 110 at core 109. OCDD 101 may alternatively contain a carbon dioxide sorbent or a combination of oxygen and carbon dioxide sorbent. OCDD 101 may be comprised of a disposable housing having a series of hollow fibers and/or gas-permeable films or fibers 115 (or membranes) that are oxygen, carbon dioxide, or oxygen and carbon dioxide permeable. Optionally, housing 104 also has a vent 114 for air to enter when draining the device after completing the depletion process to allow maximal RBC recovery.

O/CD sorbent 110 may be a mixture of non-toxic inorganic and/or organic salts and ferrous iron or other materials with high reactivity toward oxygen, carbon dioxide, or oxygen and carbon dioxide. O/CD sorbent 110 may be made from particles that have significant reaction capacity for O₂ (*e.g.*, more than 5 ml O₂/g) and can maintain the inside of OCDD 101 to less than, *e.g.,* 0.01% which corresponds to pO₂ less than 0.08 mmHg. O/CD sorbent 110 may be either free or contained in an oxygen permeable enclosure, container, envelope, etc. For example, oxygen scavengers and carbon dioxide scavengers are provided by Multisorb Technologies (Buffalo, NY), or MGC (New York, NY). Oxygen sorbents may exhibit a secondary functionality of carbon dioxide scavenging. Carbon dioxide scavengers include metal oxides and metal hydroxides. Metal oxides react with water to produce metal hydroxides. The metal hydroxide reacts with carbon dioxide to form water and a metal carbonate. Such materials can be blended to a desired ratio to achieve desired results.

In certain aspects, OCDD 101 of the present disclosure is configured to throughput and deplete approximately 100 mL of oxygen from a unit of blood. Alternatively, after passage of RBCs through OCDD, the oxygen saturation levels are reduced to less than 3 Torr in the RBCs. Alternatively, carbon dioxide levels are depleted in the RBCs to levels of approximately 10 Torr.

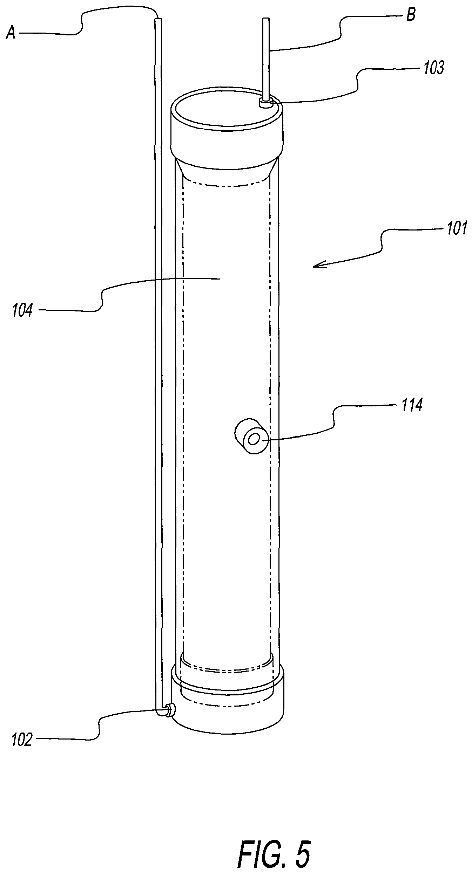

Again referring to FIGS. 5 and 6, hollow fibers and/or gas-permeable films or fibers 115 may be constructed as membranes in a flat sheet form. Hollow fibers and/or gas-permeable films or fibers 115 may be non-porous materials that are capable of high O/CD permeability rates (polyolefins, silicones, epoxies, polyesters etc) and membrane are hydrophobic porous structures. These may be constructed of polymers (polyolefins, Teflon, PVDF, polysulfone) or inorganic materials (ceramics).

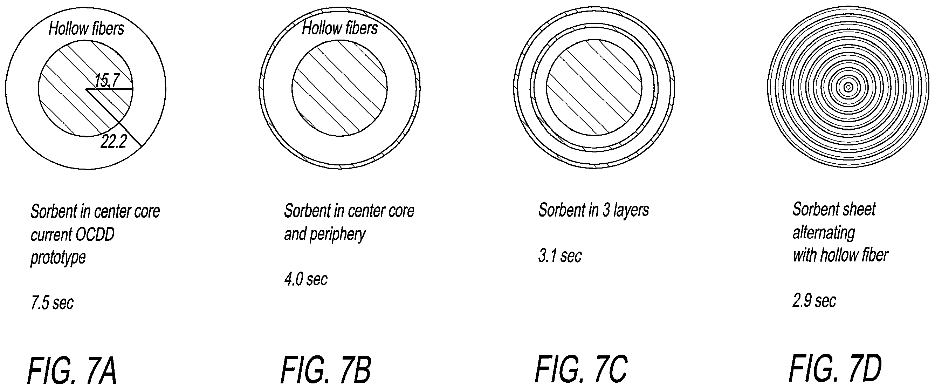

Referring to FIGS. 7B through 7D, alternative OCDD configurations (cross sectional views) are shown, with alternating sorbent 110 with hollow fibers 115 (device of FIG. 6 is represented in FIG. 7A). In the device of FIG. 6 and FIG. 7A, the characteristic diffusion time of oxygen is approximately 7.5 seconds. The placement of sorbent material relative to hollow fibers is critical because the diffusion time is proportional to the inverse square of distance from sorbent to the fiber. If the distance between the sorbent and fiber is reduced by one half the diffusion time is reduced by one quarter.

**Table 2**

| Example Specification | Eternal Gas Pathways | Eternal Gas Pathways |
|---|---|---|
| Example Serial #: | Device 70 | |
| Fiber Type: | Celgard 200/150-66FPI | Celgard 200/150-66FPI |
| Number of Fibers/unit: | 5000 | 5000 |
| Active Length of Fibers (cm): | 13 | 28 |
| Fiber OD (microns): | 200 | 200 |
| Fiber ID (microns): | 150 | 150 |
| Total Length of Fibers | 15 | 30 |
| Active Fiber Surface Area (m²): | 0.4084 | 0.8796 |

In the oxygen/carbon dioxide depletion devices disclosed herein, a plurality of gas permeable films/membranes may be substituted for the plurality of hollow fibers/films. The films and fibers may be packed in any suitable configuration within the cartridge, such as linear or longitudinal, spiral, or coil, so long as they can receive and convey red blood cells.

The lowest oxygen saturation is achieved using devices in which the sorbent is placed close to fibers to enable rapid diffusion time. Additional factors that increase oxygen and/or carbon dioxide diffusion are larger active surface area of fibers exposed to sorbent materials.

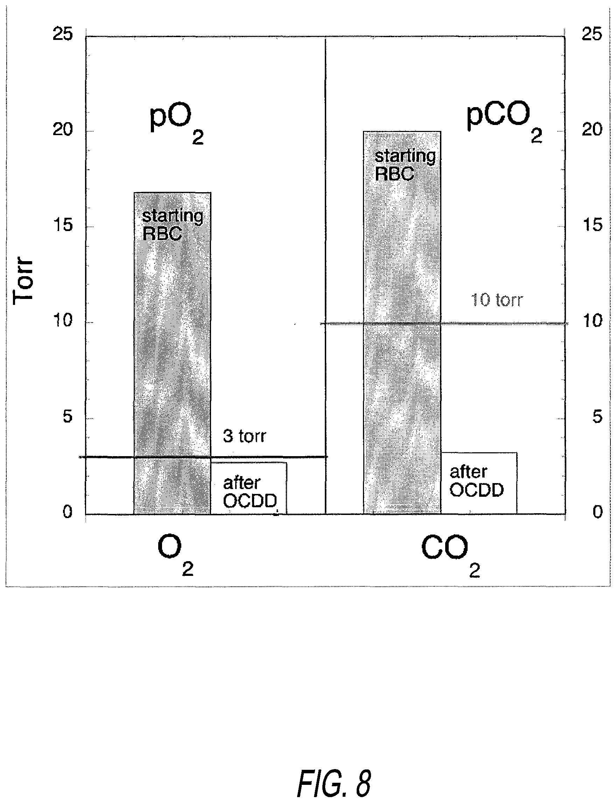

Referring to FIG. 5 and FIG. 8, the graph shows the effect of OCDD 101 on the partial pressure of RBCs that pass therethrough. At point A, prior to entry in OCDD 101, the partial pressure of oxygen in the RBCs is 16.8 Torr and at point B, the partial pressure of oxygen in the after oxygen and carbon dioxide depletion device is approximately 3 Torr. The partial pressure of carbon dioxide is approximately 20 Torr at point A and the partial pressure after RBCs pass through OCDD is approximately 3 Torr.

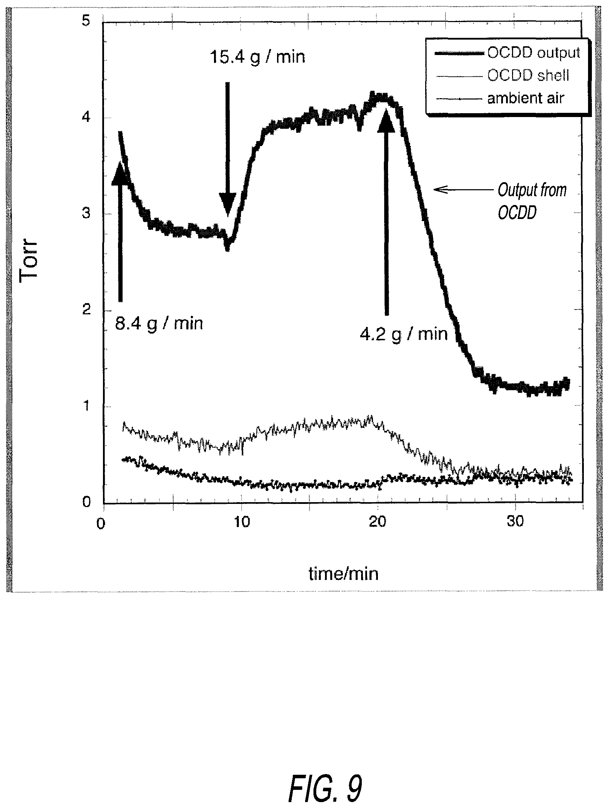

FIG. 9 shows the partial pressure of oxygen in RBCs as a function of mass flow rate of the RBCs through OCDD 101. The partial pressure of oxygen in the gas surrounding hollow fiber as measured from the vent port 114 ranges from 1 to .5 Torr depending on the flow rate of the RBCs therethrough. FIG. 8 reveals the oxygen depletion that is provided by OCDD 101. The oxygen sensor at the outlet is enclosed in a bag flushed with nitrogen gas to increase the sensitivity of the pO₂ measurement of blood; pO₂ surrounding the sensor is shown as 'ambient air' in FIG. 9.

The OCDD functions to deplete oxygen from RBCs from oxy-hemoglobin because more than 99% of such oxygen is hemoglobin-bound in venous blood. Preferably, the degree of oxygen saturation is to be reduced to less than 3 Torr within 48 hours of blood collection. The oxygen depletion is preferably accomplished at room temperature. The affinity of oxygen to hemoglobin is highly dependent on the temperature, with a partial pressure of 26 Torr at 37° C dropping to ~4 Torr at 4° C. Furthermore, this increase in O₂ affinity (Kₐ hemoglobin - oxygen binding constant) is mainly due to reduction in O₂ release rate (k_{-off}), resulting in an impractically low rate of oxygen removal once RBC is cooled to 4° C. Thus, it places a constraint on oxygen stripping such that it may be preferable to accomplish it before RBC are cooled to storage temperatures of 1° C to 6° C.

In addition to oxygen depletion, carbon dioxide depletion has the beneficial effect of elevating DPG levels in red blood cells. Carbon dioxide exists inside RBCs and in plasma in equilibrium with HCO₃⁻ ion (carbonic acid). Carbon dioxide is mainly dissolved in RBC/plasma mixture as carbonic acid and rapid equilibrium between CO₂ and carbonic acid is maintained by carbonic anhydrase inside RBC. Carbon dioxide is freely permeable through RBC membrane, while HCO₃⁻ inside RBC and plasma is rapidly equilibrated by anion exchanger (band 3) protein. When CO₂ is removed from RBC suspension, it results in the known alkalization of RBC interior and suspending medium. This results from removal of HCO₃⁻ inside and outside RBC; cytosolic HCO₃⁻ is converted to CO₂ by carbonic anhydrase and removed, while plasma HCO₃- is removed via anion exchange inside RBC. Higher pH inside RBC is known to enhance the rate of glycolysis and thereby increasing ATP and DPG levels. ATP levels are higher in Ar/CO₂ (p<0.0001). DPG was maintained beyond 2 weeks in the Argon purged arm only (p<0.0001). Enhanced glycolysis rate is also predicted by dis-inhibition of key glycolytic enzymes via metabolic modulation and sequesterization of cytosolic-free DPG upon deoxygenation of hemoglobin as a result of anaerobic condition. DPG was lost at the same rate in both control and Ar/CO₂ arms (p=0.6) despite thorough deoxygenation of hemoglobin, while very high levels of ATP were achieved with OFAS3 additive.

By depleting carbon dioxide in the OCDD, the pH of RBCs in cytosol is increased. Further, 2, 3-DPG levels are increased for the first 3 weeks of storage and ATP level is maintained at high levels. These factors enhance the viability of RBCs prior to being stored at oxygen depleted storage in Phase B.

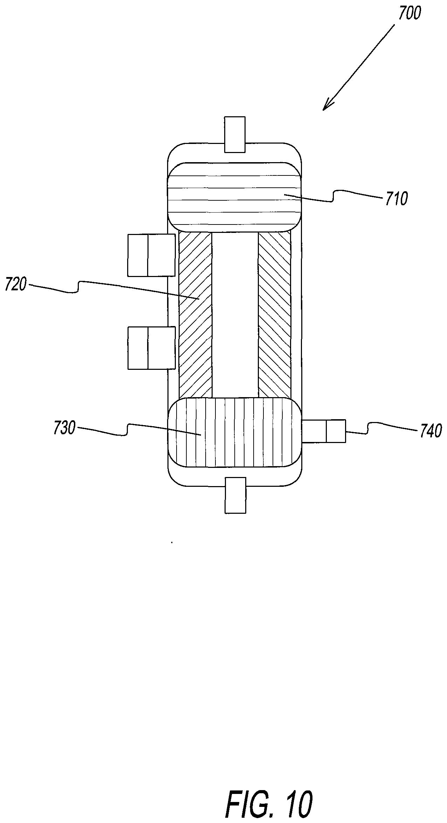

Referring to FIG. 10, a further instance of OCDD device 750 is shown, in which flow of oxygen free gas or oxygen free gas with carbon dioxide through the body of the device (two ports protruding on the left side of cylinder) can be used to combination with a leukoreduction filter 710, OCDD 720, a plasma separator 730. Multifunction OCDD 750 eliminates the need for centrifugation of the whole blood, received from donor 15, which is currently a necessity by using a centrifuge. By combining these three devices into a single device, the need for a separate centrifuge, a highly costly and cumbersome device, is eliminated. Plasma flows through port 740 to a further collection bag for further processing. Accordingly, in this instance, whole blood can be collected from a donor, leukocytes can be removed, oxygen and or carbon dioxide can be removed and plasma and platelets can be removed to pass RBCs through device. The RBCs are then deposited into collection bag 200 after additive solution is added through the device for storage or transfusion to a recipient. Multifunction OCDD 750 as part of collection system 10 and system 100 permit rapid transformation of whole blood to stored RBCs for immediate storage or transfusion to a recipient.

### Editing

Before oxygen and/or carbon dioxide are removed from the RBCs, the whole blood or RBCs may be edited. Editing RBCs is the process of identifying and removing blood cells that have a poor likelihood of surviving the transfusion process or will likely die shortly after transfusion. Editing moribund RBCs, or dead or dying red blood cells, may be employed by using, for example, a filter-like editing device 500. Editing can occur at various times during the storage process, *e.g.,* as shown in FIG. 1. For example, editing can occur with whole blood or RBCs, before being O/CD depleted and prior to storage in an anaerobic storage environment. While FIG. 1 shows the editing step being performed by editing device before oxygen and or carbon dioxide have been removed, editing can alternatively be performed at different stages of the storage process. For example, editing can be performed immediately before transfusion after storage in storage bag 200.

Editing can be important because a leading cause of morbidity and mortality to transfused patients is the non-viable portion of the blood that is transfused independent of any pathogen transmission. RBCs that are compromised or that will be removed by the spleen by the reticuloendothelial system shortly after transfusion may threaten to overwhelm the already compromised recipient. Up to 25% of transfused cells are removed by recipient in the first twenty four hours after transfusion. These removed cells are harmful because they contribute immediately to the excess iron burden of the recipient, which may be a critical parameter for chronically or massively transfused patients. Also, these cells may cause capillary blockage due to reduced deformability or aggregate formation, leading to poor tissue perfusion and even organ failure. Thus, substantial benefits are expected if one can remove these less viable RBCs prior to transfusion.

There are several techniques that may be used to edit the red blood cells. The first technique is a centrifugation process to separate old and young RBCs before storage based on characteristic buoyancies of young and old RBCs.

A second technique applies a biomechanical stress, such as an osmotic shock, to hemolyze weak cells before or after storage in combination with a buffer exchange step. The applied biomechanical stress immediately identifies those cells that are weak to rapidly contrast with the stronger RBCs to enable mechanical separation. The weak RBCs are those that contribute to recipient morbidity and mortality, particularly, with individuals with already compromised or overloaded immune systems. Up to 25% of RBCs that arrive to a recipient are already dead and can have deleterious effects on the recipient. By editing the RBCs, that number can be reduce by 50% to 75%.

A third technique applies to the deformability of the RBCs. Bump array microfluidic devices containing staggered pillars (Huang, L.R., et al., "Continuous particle separation through deterministic lateral displacement,". Science 304(5673): 987-90 (2004)), allow deformable RBCs to pass through the pillars while deformable RBCs can not pass through the pillars and are bumped into separate channels.

A further technique for editing the RBCs uses a filter system to remove RBC exhibiting a specific surface marker. RBCs exhibiting known surface markers such as phosphatidiyserine or aggregated protein 3 can be trapped by a filter surface modified with high affinity ligand (e.g., Annexin IV or antibodies against specific surface marker protein).

An additional technique uses the same high affinity ligands in the second technique, conjugated to make a multimeric molecule such that RBCs exhibiting target surface markers forms aggregate. This can then separated by filtration or centrifugation.

### Irradiation

A further processing step that RBCs may be subjected to is irradiation, e.g., by either gamma or X-ray radiation. The presently claimed method comprises irradiating packed RBCs with gamma or x-ray irradiation, wherein irradiation occurs before, during, or after storing, wherein irradiation before storing occurs after reducing oxygen and carbon dioxide. Irradiation of RBCs is of significance to avoid transfusion related complications. Transfusion-associated graft-versus-host disease (TA-GVHD) is a rare but nearly always fatal complication associated with transfusion therapy in severely immuno-compromised blood recipients (for example, bone marrow transplant recipient, patients receiving aggressive chemotherapy, premature neonates). Prevention of TA-GVHD requires complete removal of, or cessation of the proliferative potential of T-lymphocytes from donor blood. For instance, leukoreduction filters are not adequate in prevention of TA-GVHD because leukoreduction filters may not completely eliminate lymphocytes. Thus, lymphocyte inactivation by X-/gamma-irradiation may be preferred for TA-GVHD prevention.

Gamma-irradiation abrogates proliferation of T-lymphocytes by damaging the DNA directly and via reactive oxygen species (ROS), namely hydroxyl radicals produced during gamma-radiolysis of water. Although RBCs do not contain DNA, ROS generated by gamma-irradiation have been shown to cause significant damage to the RBCs. The major damage observed includes: i) increased hemolysis; ii) increased K+ leak; iii) reduction in post-transfusion survival; and iv) reduced deformability. Such damage is similar to, but an exaggerated form of storage-induced damage of RBC. The compromised status of RBC is well known to the physicians who administer such compromised RBCs, and FDA mandates restricted use of such RBCs in terms of shortened shelf life after gamma-irradiation (14 days) and/or 28 days total shelf life for irradiated units.

The irradiation of blood components has received increased attention due to increasing categories of patients eligible to receive such blood to prevent transfusion-associated graft versus host disease. However, irradiation also leads to enhancement of storage lesions, which could have deleterious effects when such blood is transfused. It is well known in the field that the main deleterious side-effect of radiation on RBC is oxidative damage caused by ROS.

Radiation damage to RBC in the presence of oxygen can occur in two ways;
i) By ROS generated during and immediately after irradiation. ROS can attack proteins and lipids in vicinity, as well as to initiate peroxidation cycle of lipid and protein using oxygen to fuel.
ii) Met-Hb and its denaturation products generated in i) above act as catalysts to further cause ROS-mediated oxidative damage during subsequent refrigerated storage of RBC. This is an enhanced version of storage lesion development using O₂.

ROS is a major culprit in causing deterioration of RBCs during refrigerated storage at blood banks. Storing RBCs under anaerobic conditions significantly reduces such damages caused by ROS. Accordingly, deleterious or negative effects of gamma- and X-ray irradiation steps to RBCs, are substantially offset by the protective benefits oxygen and or carbon dioxide removal and the subsequent anaerobic storage. Therefore, irradiation of RBCs is preferably performed after oxygen removal in OCDD 100 of FIG. 2. Additionally, gamma irradiation and X-ray irradiation can occur when RBCs are stored in storage bag 200 or subsequent to storage prior to oxygen addition before transfusion.

While oxygen, carbon dioxide or oxygen and carbon dioxide removal take place before irradiation, if irradiation occurs before oxygen removal (FIG.1), oxygen removal should take place within twenty four hours thereafter to limit effects of on RBCs, although this does not form part of the invention.

### Pathogen Inactivation

After RBCs have been collected, treatment for the removal of infectious agents that may be present in donor blood and potentially passed to recipient 50 receiving transfusions, may be effected. Infectious agents include microorganisms such as viruses, especially retroviruses, bacteria, fungi and non-microbial agents such as self-replicating proteins (prions) and nucleic acids. A process called pathogen inactivation or reduction removes such dangerous infectious agents from the RBCs. However, the chemical processes of pathogen inactivation or reduction are also potentially damaging to the RBCs.

A pathogen inactivation process may involve chemical and light or riboflavin and light therapy. In general, pathogen inactivation or reduction may be performed before storage in the anaerobic storage environment for whole blood, and for RBCs that have been separated from whole blood, before passage through OCDD or after passage through OCDD and before storage. The presently claimed method comprises inactivating pathogens in packed RBCs with riboflavin and light therapy after removing oxygen and carbon dioxide.

Since RBCs are stored in an anaerobic environment, growth of aerobic bacteria and parasites are prevented. Bacteria such as *Yersinia enterocolotica*, *Serratia liquefaciencs* and *Staphylococcus* strains are anaerobic and require oxygen for growth and multiplication. Parasites such as *Plasmodium falciparum* (malaria protozoan), *babsea* (babesiosis) and *Trypanosoma cruzi* (Chagas disease) are documented infections in the US following blood donations These microaeophilic organisms and are exposed to varying oxygen concentrations during their life cycle. They survive well in reduced oxygen environments, but may not adapt to the anaerobic conditions developed during RBC storage.

Due to the reduction of ROS production under anaerobic state, gamma or X-ray may be used at increased dosage and/or time compared to T cell inactivation for pathogen inactivation.

### Blood Storage Bag (not according to the invention)

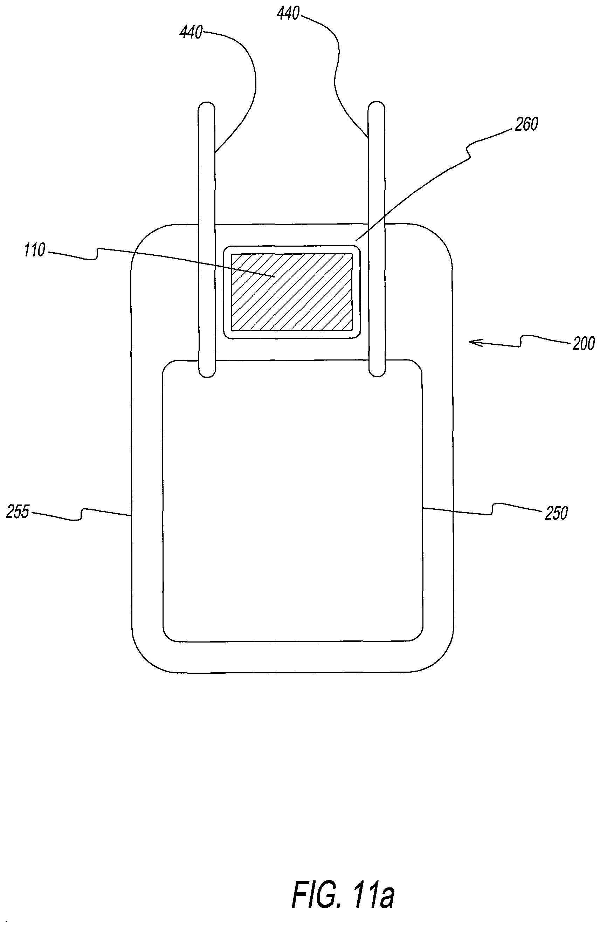

Referring to FIGS. 2 and 11a, a blood storage bag 200 according to an instance of the present disclosure is provided. Blood bag 200 has an inner blood-compatible bag 250 (preferably polyvinyl chloride (PVC)), and an outer barrier film bag 255. The material of bag 250 is compatible with RBCs. Disposed between inner bag 250 and outer oxygen barrier film bag 255 is a pocket that contains a sorbent 110. Barrier film bag 255 is laminated to the entire surface of inner bag 250. Sorbent 110 is contained in a sachet 260, which is alternately referred to as a container, enclosure, envelope, pouch, pocket, etc. Sorbent 110 is optimally located between tubing 440 that leads into and from bag 200 and port 415, and specifically between inner bag and outer oxygen barrier film bag 255. This location will ensure that oxygen disposed between these two bags will be scavenged or absorbed. Sorbent is ideally located in a sachet 260 and not in contact with RBCs. Sorbent may include oxygen sorbents, and may also be combined with carbon dioxide sorbents, enabling sorbent 110 to deplete both oxygen and carbon dioxide at the same time.

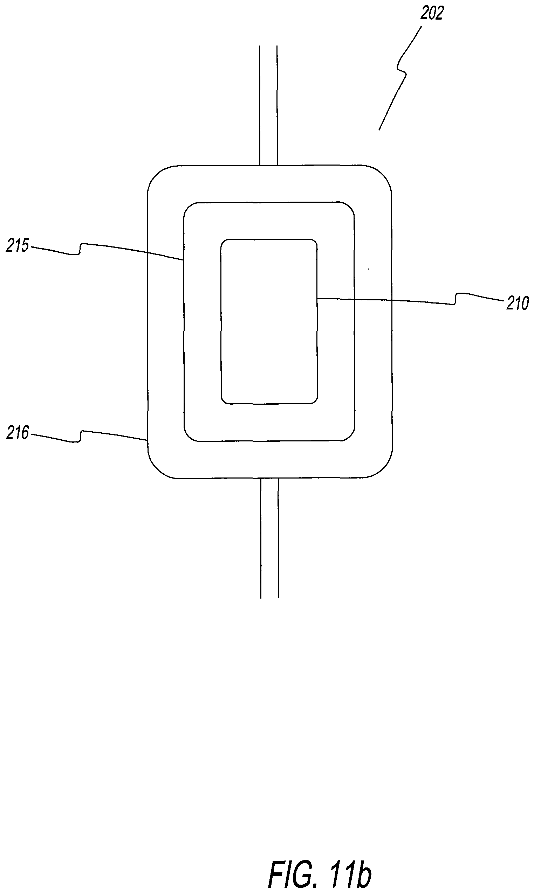

Referring to FIG. 11b, storage bag 202, is similar to bag 200; however, bag 202 is a laminated bag. Bag 202 has a inner PVC blood bag 210, an outer barrier bag 216, and a sorbent layer 215 between blood bag 210 and outer barrier bag 216.

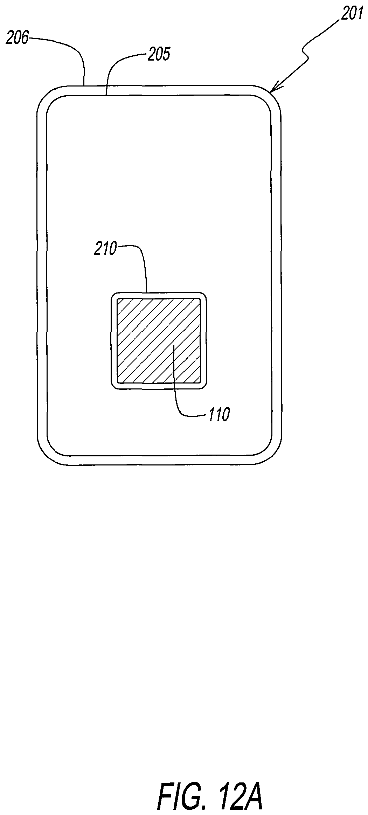

In FIG. 12A, a small sachet 210 contains sorbent 110. Small sachet 210 is enclosed inside of PVC bag 205 and is preferably made from a silicone or siloxane material with high oxygen permeability of biocompatible material. Sachet 210 has a wall thickness of less than 0.13 mm thickness ensures that O₂ permeability ceases to become the rate-limiting step. PVC bag 205 may also contain carbon dioxide sorbent. Again, sorbent 110 may be an oxygen, carbon dioxide and oxygen and carbon dioxide sorbent.

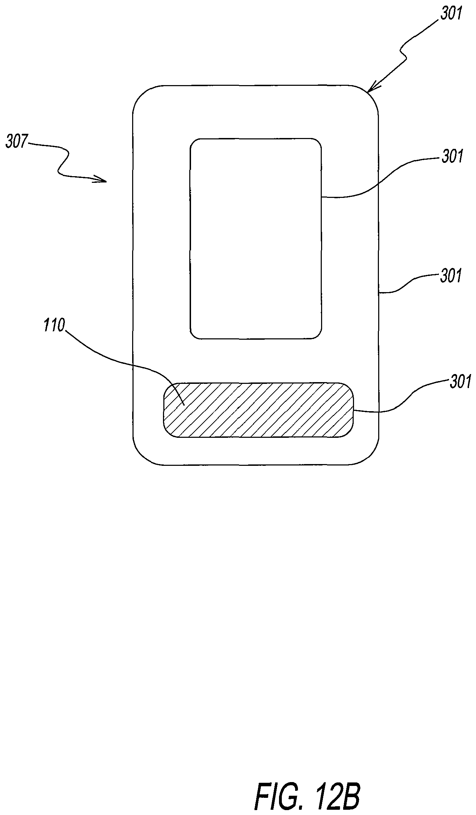

In FIG. 12B, RBCs are stored in storage bag 307 that has a secondary bag 301 in order to maintain an anaerobic storage environment for RBC storage. Secondary bag 301 may have a transparent oxygen barrier films (e.g., nylon polymer) that compensate for the inability of PVC blood bag 305 operate as a sufficient oxygen barrier to maintain RBCs in an anaerobic state. Secondary bag 301 may be made with an oxygen barrier film, preferably a nylon polymer or other transparent, flexible film with low oxygen permeability. Bag 307 contains a sorbent sachet 310 containing a sorbent 110 that is an oxygen, carbon dioxide or oxygen and carbon dioxide sorbent.

Referring to FIGS. 12A and 12B, blood storage bags 201 and 301 are configured to store RBCs for extended storage periods of time in an anaerobic storage environment. Inner blood storage bags 205 and 305 are preferably made from DEHP-plasticized PVC and are in contact with RBCs. DEHP-plasticized PVC is approximately 200 fold less permeable to oxygen compared to silicone. Inner storage bags can also be made from non DEHP plasticized PVC or other non DEHP plasticized polymer. DEHP has a protective effect on the RBC membrane, but this effect is unnecessary when the RBCs are stored anaerobically.

However, PVC is insufficient as an oxygen barrier to maintain the anaerobic state of RBCs throughout the storage duration. Therefore, blood storage bags 201 and 301 may be fabricated with outer transparent oxygen barrier film 206, 306 (*e.g.* nylon polymer, aluminum oxide coated nylon *etc.*) laminated to the outer surface inner blood bag 205 and 305. This approach, as well as one shown in Fig. 1, uses accepted plastic materials for blood contact surface (for case of DEHP/PVC, supplying DEHP for cell stabilization) at the same time prevents oxygen entry into the bag during extended storage.

Alternatively, transparent organic oxygen sorbent film may be laminated between 205/206 or 305/306 in place of 210/110 or 310.

OCDD 101 and various storage bags of the present disclosure can be used in varying combinations. For example, OCDD 101 of FIG. 1 can be used with blood bag of FIGS. 11, 11b, 12A or Fig.12B. Other combinations and configurations are fully within the scope of the present disclosure.

During storage in bag 200 different components may be added to RBCs stored anaerobically and during carbon dioxide depletion. In addition to additives, metabolic supplements may also be added to red blood cells. Metabolic supplements can be provided to RBCs specified times and rates or frequencies by a metering device placed within the main storage bag, or added through pre-connected PVC bags. Metabolic supplements are added to RBCs during storage at 4 °C. Red blood cell storage extends well beyond the current 6-week limit for 12 or up to 20 weeks at 4 °C, with levels of 2-3 DPG and ATP that are above those found in freshly drawn blood. The metabolic supplement includes pyruvate, inosine, adenine, and optionally dibasic sodium phosphate and/or monobasic sodium phosphate. Additionally, nutrient supplementation may optionally include supplements that provide antioxidants to the storage medium, including, but not limited to analogues of reduced glutathione, vitamin C and vitamin E. Current refrigeration storage technology is essentially a premature aging process of RBCs in contrast to the metabolism protection system for use in the present disclosure. Current refrigerated storage of red blood cells does not maintain appropriate cellular glutathione levels. Glutathione supplementation may extend the storage time of RBCs. The amounts and timing of glutathione supplementation may conveniently be determined and optimized as necessary.

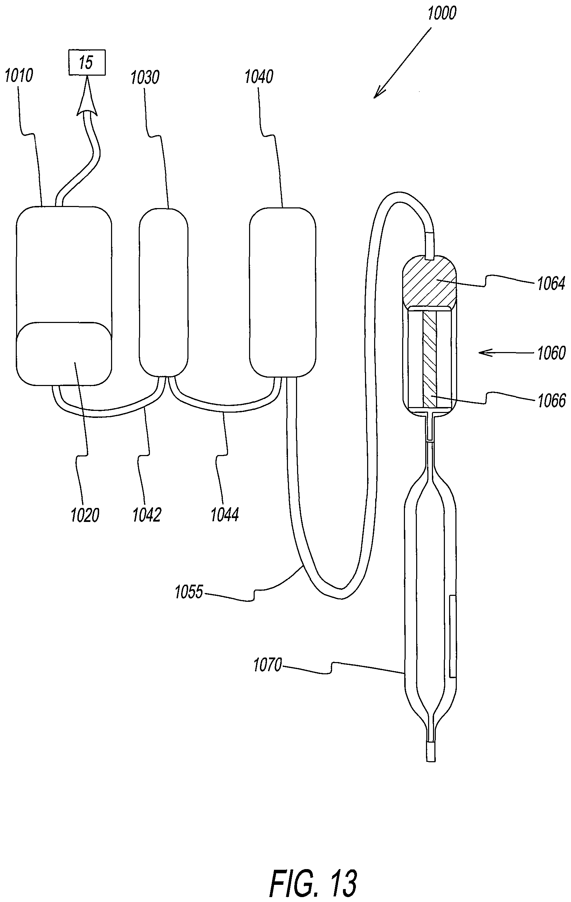

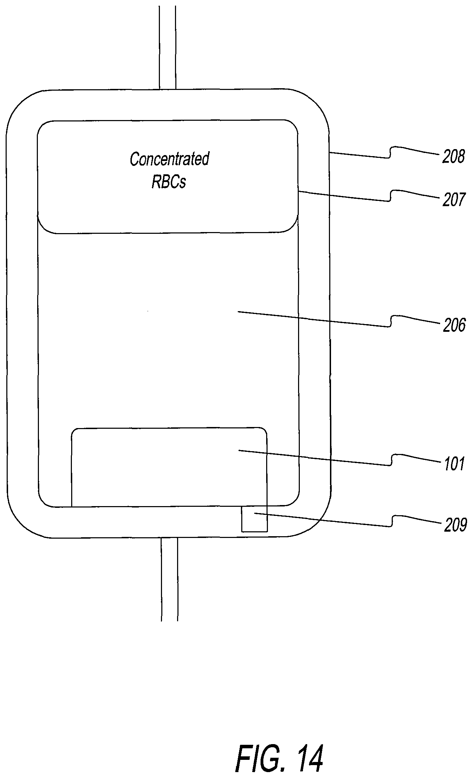

Referring to the drawings and in particular to FIG. 13, another instance of a disposable blood anaerobic storage system is shown and referenced using reference numeral 1000. The blood storage system includes a blood collection bag 1010, a combination oxygen/carbon dioxide depletion device 1060 that includes a leukoreduction filter 1064 and a oxygen and/or carbon dioxide depletion portion 1066, and an anaerobic blood storage bag 1070. System 1000 also includes a collection bag 1030 for liquid plasma and/or platelets. Combination OCDD 1060 not only removes oxygen and carbon dioxide from red blood cells traveling therethrough, but also filters excessive white blood cells from the red blood cells. This instance of FIG. 14 offers a single-use, disposable, low cost system. Tubing connects the various components of the blood storage system 1000. Tube 1042 connection bag 1010 connects collection bag 1010 and bag 1030 and tubing 1044 connects bag 1030 and bag 1040. Tube 1055 connects combination OCDD 580 with additive bag 1040.

The system for use in the present disclosure may recognize that blood cells in storage continue to metabolize. It is desirable to reduce their metabolic rate as low as possible over time of storage, and yet maintain healthy viable cells that are of high quality for transfusion. The present disclosure may uniquely protect essential metabolism, prolongs the shelf life of refrigerated erythrocytes, and provides high quality blood product. Further, refrigeration reversibly disables the enzymes essential for met-Hb reduction *in vivo,* increases the solubility of damaging O₂ (almost by a factor of 2) in the environment of the red blood cells, and permits the level of ATP to decrease by diminishing the glycolytic rate (at 4 °C. the rate is about 1% of that found at 37 °C.). Reduction of red cell ATP concentration results in echinocyte (i.e. an unstable form of red blood cells) formation, increased rates of membrane vesiculation, loss of red cell surface area, and accelerated sequestration by splenic macrophages. Vesiculation continues throughout the cold storage period, is exacerbated by echinocyte formation, and decreases red blood cell survival by decreasing red blood cell membrane area.

Oxygen removal can be conducted at any temperature that maintains good viability of the RBC. Preferably, oxygen is removed between about 1°C and about 37 °C provided that RBC viability is maintained. Once in a blood storage device of the present disclosure, the RBC can be stored under refrigeration in the manner consistent with common industry practice for storage of blood products, preferably at a temperature between 1 °C and 10 °C, and more preferably at about 4 °C. Such storage periods range from about 6 to about 20 weeks and longer. Preferred storage periods are about 6 to about 15 weeks duration or longer provided RBC quality is maintained.

### Pre-Transfusion

Prior to transfusion of RBCs stored according to the invention to a patient or recipient, various processes can be affected to maximize acceptance of RBCs by the recipient and to optimize the condition of the RBCs.

In those patients who are either small or whose circulatory systems cannot process a great influx of RBCs, the volume of the RBCs must be reduced immediately prior to transfusion. Such patient who may face such an issue include those suffering from congestive heart failure or neonates. Volume reduction can be accomplished using a variety of methods.

When RBCs are stored for a length of time, the RBCs may generally be stored in a storage bag, such as bags of FIGS. 11a, 11b 12A and 12B. In some aspects, storage bags can have a hydrophilic membrane compartment in the top 1/2 of the bag, such as that of bag 208 of FIG.14. In an aspect, bag 208 may have a hydrophilic membrane 207 having a membrane pore size must be less than < 4 micron to retain the RBCs cells and to prevent them from flowing through. When membrane 207 filled with a concentration of RBC with low hematocrit, plasma and additive solution will pass through the membrane into the lower compartment 206 concentrating RBCs in membrane 207. The top portion of the lower compartment needs a check valve 209 so the fluid will not escape during transfusion. Bag 208 may have a sorbent 101, as discussed above for purposes of continued depletion of oxygen, carbon dioxide, and oxygen and/or carbon dioxide.

More conventionally, a portion of RBC of low hematocrit can flow into a small hollow fiber/film device having hydrophilic fibers/films, such as the fibers/films of OCCD 100. A portion of the RBC will flow into the fiber/film lumens and liquid and the liquid portion will pass through the fiber/film wall. A differential pressure across the fiber/film wall will be used to control RBC and fluid flow. This is another method of concentrating the RBCs in advance of transfusion.

Alternatively, RBCs may be concentrated by passage through several microfluidic chips that use the inertia of the RBCs. The microfliudic chips harness the inertia of the RBCs by forcing the RBCs to flow through a plurality of narrow channels such that only one cell is able to pass through each of the plurality of channels at a time. The cells are in the center of the channel, they exit through a center outlet port, and fluid, plasma and additives can exit in ports adjacent to the center port. The microfluidic chip may be scaled up for volumes. Microfluidic chips may contain at least one network unit disposed in a substrate. Microfluidic devices have an aspiration pressure to enables movement of RBCs through the network unit.

A further processing step that is necessary immediately prior to transfusion is the introduction of nitric oxide (NO) to the RBCs to enhance vasoregulatory function. There is increasing awareness that blood transfusion using banked blood is not only providing fully perceived benefits, but in some cases, harmful to some recipients. One of the major reasons behind lower-than-expected efficacy of transfused blood is postulated to be the loss of vasoregulatory function of RBC caused by degradation of nitric oxide (NO) sequestered in hemoglobin (Hb) molecules within RBC. A recent report showed that as short as 3 hoursafter blood collection, NO in RBC was lost, and its vasoregulatory activity can be restored with addition of NO replenishing compounds. Accordingly, the introduction of NO to RBCs during storage in blood bag 200, immediately prior to transfusion and after storage will assist the recipient in receiving optimal benefits from the transfusion. Because of increased stability of NO in anaerobic conditions, nitric oxide is added to the anaerobic environment of storage bag 200 prior to transfusion, for example. Additionally, NO can be added in the post-storage phase C prior to the addition of oxygen before transfusion. NO addition requires prior oxygen removal due to its inherent instability in the presence of oxygen. Additionally, NO must be added immediately before transfusion in the form of NO gas, NO precursor reagents, or nitrite. NO can be added to RBCs in storage bag 200 using a small bag or cartridge to inject above materials in form of a gas or nitrate or other precursor chemical as part of a transfusion set.

Immediately before transfusion, oxygen can be supplied to RBCs to oxygenate RBCs. Addition of oxygen must be accomplished during post-storage phase C after gamma and x-ray irradiation and nitric oxide addition, preferably immediately before transfusion at the bedside. The presence of oxygen with the processes of gamma and X-ray irradiation and the addition of nitric oxide are deleterious to the RBCs as discussed above.

The benefits of oxygen removal and or carbon dioxide removal from RBCs before storage in combination with and other therapies has a positive effect on the outcome of the stored RBCs in advance of transfusion.

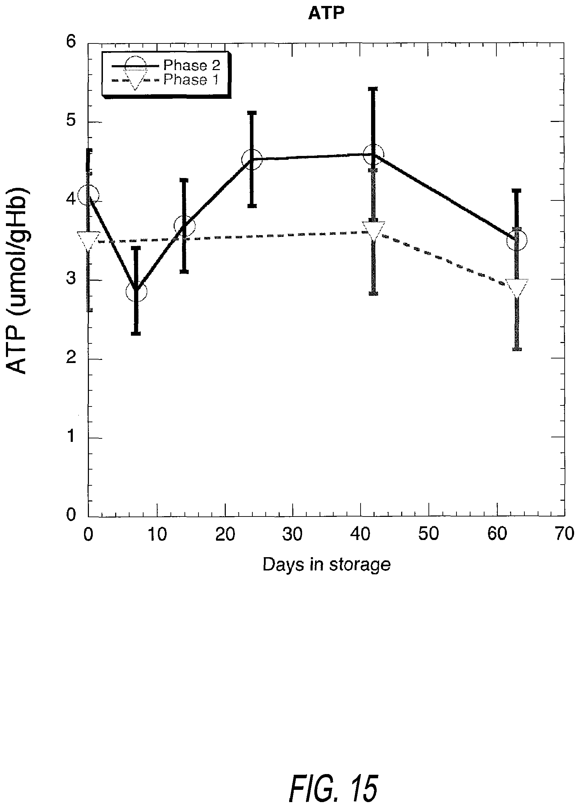

FIGS. 15, 16 and 17 show the benefits offered by the proposed storage system and process of the present disclosure. In Phase I, represented by dotted line, RBCs are flushed with an inert gas to remove oxygen and are stored in an anaerobic canister for 9 weeks. In Phase 2, represented by solid line, RBCs are oxygen, carbon dioxide, or oxygen and carbon dioxide depleted in OCDD 100 and are stored in anaerobic storage bag 200 for 9 weeks. Phase 2 shows that ATP levels of RBCs are significantly higher at weeks 3 through 9. By maintaining high levels of ATP, RBCs maintain the ability of dilate the pre-capillary arteriole maintain a high metabolic level. Further, ATP can be boosted and significantly stimulated during the first four weeks of storage by initially depleting oxygen levels and maintaining carbon dioxide levels in the presence of a wide range of additives. FIG. 15 shows that the shelf-life of RBCs is substantially enhanced by reducing oxidative damage at 1-6 °C and maintaining high levels of ATP for an extended period.

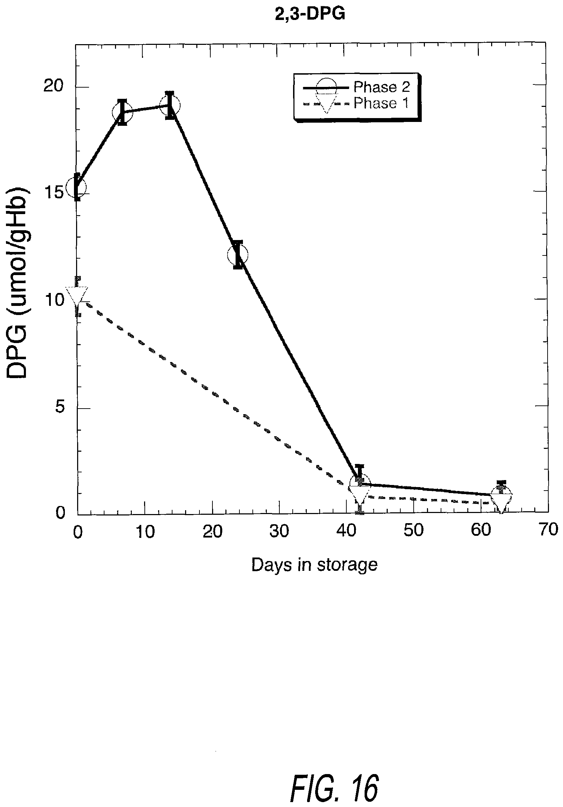

FIG. 16 shows that under Phase 2 anaerobic conditions, 2,3 DPG is maintained at a high level by depleting carbon dioxide at the onset of storage. The transfusion of high 2,3 DPG blood with full oxygen carrying capacity comparable to fresh blood provides significant benefits to patients with critical and immediate oxygen needs. The rate at which 2,3, DPG declined after week 3 is typical.

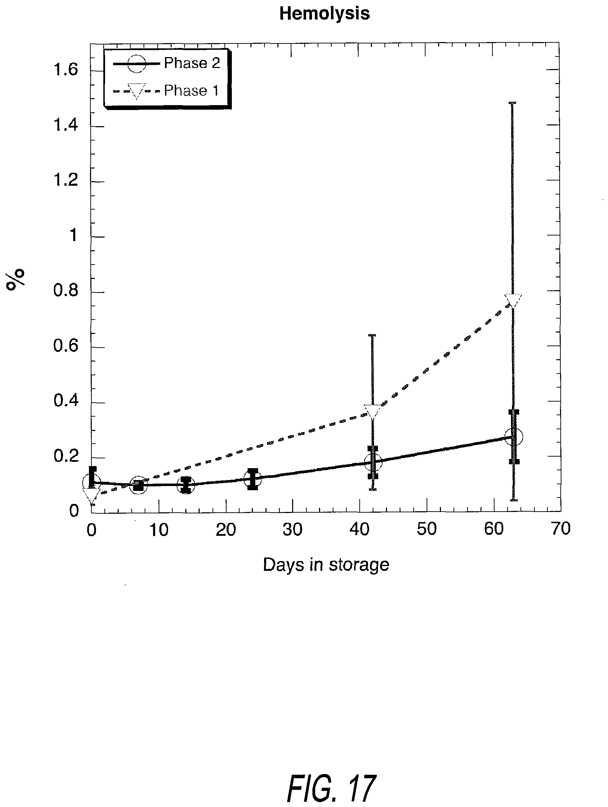

Referring to FIG. 17, hemolysis is significantly lower during Phase 2 as compared to hemolysis during Phase 1. In particular, hemolysis is significantly lower at weeks 6 through 9 of storage. Hemolysis is a concern for all transfused patients and is particularly a concern for patients under chronic transfusion therapy. Patients with inherited hemoglobinopathies such as sickle cell disease (SCD), alpha- and beta- thalassemia, require repeated periodic transfusions of 30 or more units per year. These patients' RBCs have defective hemoglobin that does not function properly in gas transport, and often have RBCs of limited life span. These patients' own RBCs, together with RBCs from chronic transfusion therapy, can overload the body's capacity for iron. Long-term iron overload is highly toxic, and the complications that arise from it become a main source of morbidity unless patients are placed under continuous iron chelation therapy. One of the major sources of excess iron for chronically transfused patients is hemoglobin originating from non-viable RBCs (as a result of accumulated storage lesions) that are destroyed immediately after transfusion. By reducing the number of non-viable RBCs, anaerobic storage of RBCs with higher 24-hr recovery reduces addition of excess iron to these patients.

RBC storage life can be measured by the extent of vesicle formation, extent of hemolysis, and total cellular ATP levels. Long storage life is obtained when the tesicle formation is low, hemolysis is low and high ATP levels are sustained, preferably above about 2-3 .mu.mol ATP per g Hb. All of these parameters may be measured by the conventional methods known to those of skill in the art. For example, samples of cells can be assayed for the extent of hemolysis by calculating the fraction of supernatant hemoglobin relative to total hemoglobin. To measure ATP levels, for example, RBCs can be assayed for ATP according to the methods described in Technical Bulletins 336-W and 35--(Sigma Chemical Co., St. Louis, Mo.).

As used herein, improved or prolonged shelf life or improved storage of RBCs refers to the preservation of viable RBCs for an extended period of time relative to the current standard of about 6 weeks. In certain instances of the present disclosure, substantial oxygen removal provides RBCs with an extended storage life of about 7-15 weeks. In other instances according to the present disclosure, substantial oxygen removal provides RBCs with an extended storage life up to 20 weeks or greater, particularly when cells are suspended in the storage solutions provided by the present disclosure. In another aspect, substantial oxygen removal provides RBCs with an extended storage life of about 10-15 weeks. In other aspects, the extended storage life may be 10 to 20 weeks or 10 to 25 weeks. In a further aspect, storage life can be prolonged by preventing 2,3-DPG feedback inhibition of the RBC glycolytic pathway.

The *in vitro* parameters measured after storage of RBCs provide a means to measure *in vivo* survival of RBCs. The conventional means to assess *in vivo* survival is to determine the percentage of cell survival 24 hours post transfusion in a recipient. Typically in the USA, the average percentage of cell survival needs to be about or better than 75% to provide an acceptable RBC product. The three parameters, vesicle production, extent of hemolysis, and ATP levels, may be routinely used individually in the art to predict *in vivo* cell survival.

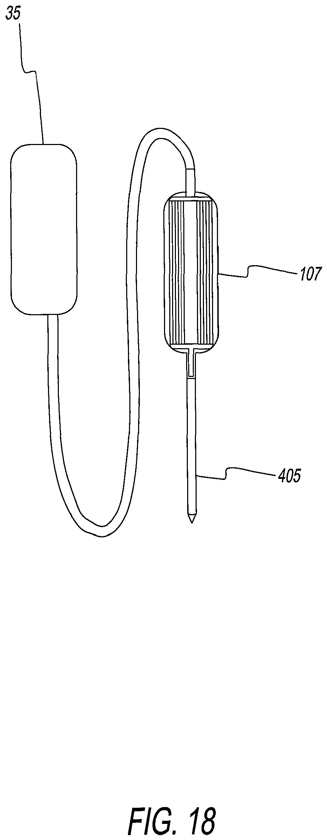

Referring to FIG. 18, immediately before RBCs are to be transfused, such RBCs can be placed in a further bag 35 in connected to a device 107 to add back oxygen prior to transfusion. Significantly, oxygen may be added back to RBCs after any gamma or x-ray irradiation or addition of nitric oxide to avoid the development of deleterious storage legions addressed above. Device 107 is like device OCDD, however, it does not have O₂/CO₂ sorbent material, but instead contains pure oxygen or air in the inner space containing hollow fibers. This use is for special cases, such as massive transfusions, where the capacity of the lung to re-oxygenate transfused blood may not be adequate, or sickle cell anemia. Once the oxygen is added back, transfusion using needle 405 can occur.

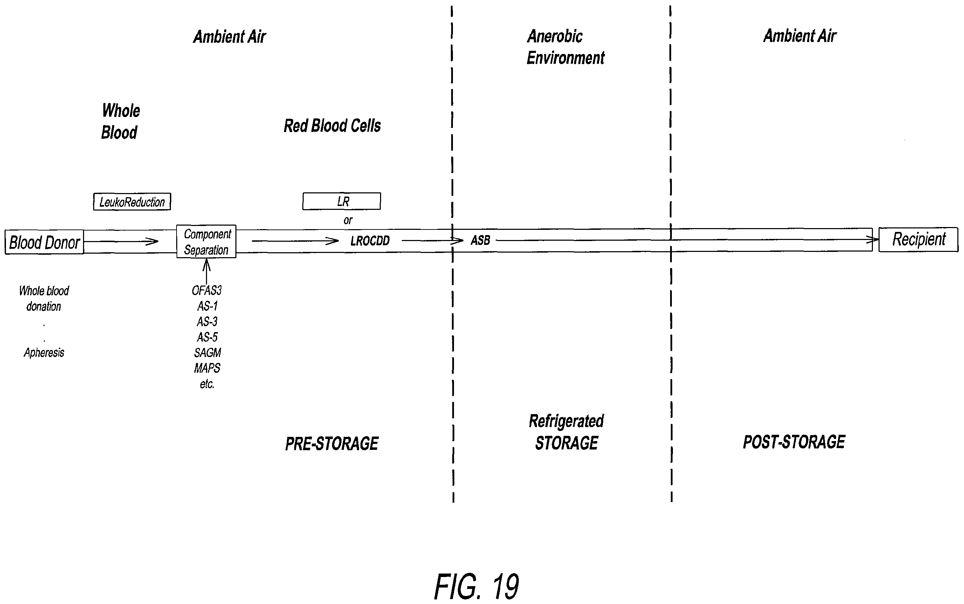

Referring to FIG. 19, a possible configuration of FIG. 1 shows oxygen depletion of RBCs and leukoreduction of RBCs prior to storage an anaerobic storage bag. FIG. 19 shows whole blood that may be obtained from a donor or by apherisis, may be separated into components of plasma, platelets and RBCs. An additive solution may be added to RBCs that are either leukoreduced prior to oxygen, carbon dioxide or oxygen and carbon dioxide depletion or leukoreduced after oxygen, carbon dioxide or oxygen and carbon dioxide depletion.

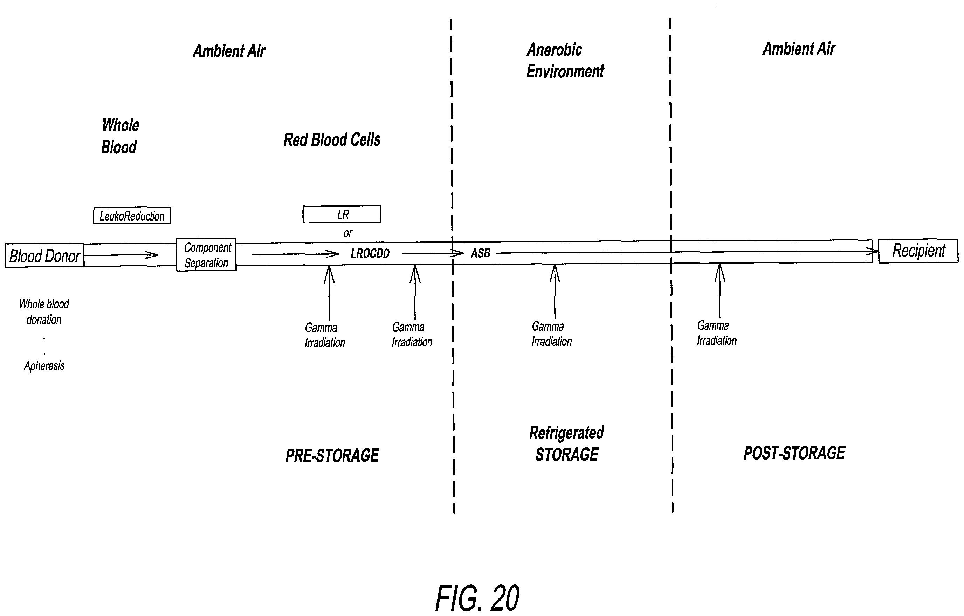

FIG. 20 illustrates a configuration of the flowchart of FIG. 1, including leukoreduction, oxygen, carbon dioxide or oxygen and/or carbon dioxide depletion. FIG. 20 illustrates the step of leukoreduction during the whole blood or after component separation at which time RBCs can be leukoreduced. Additionally, leukoreduction can be conducted using a combination leukoreduction device 1060 that is also an oxygen and carbon dioxide depletion device. Gamma irradiation or x-ray irradiation can occur at various times during Phase A, Phase B or Phase C. Gamma and x-ray irradiation can occur during anaerobic conditions after the removal of oxygen in OCDD device 100, during storage in anaerobic storage bag 200 or in post-storage phase before the addition of oxygen prior to transfusion. Irradiation is preferably performed in an anaerobic environment because an anaerobic environment minimizes oxidative damage by removing fuel of oxidative reactions. Alternatively, when gamma or x-ray irradiation occur before anaerobic conditions are present, RBCs must undergo oxygen depletion shortly thereafter and preferably within 24 hours.

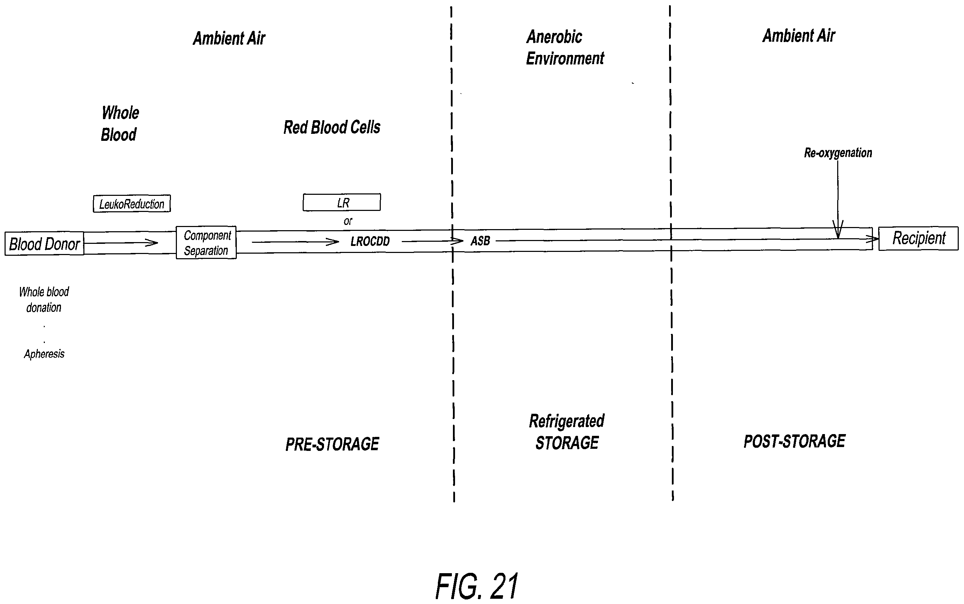

FIG. 21 illustrates a configuration of the flowchart of FIG. 1, including leukoreduction, oxygen, carbon dioxide or oxygen and/or carbon dioxide depletion and re-oxygenation immediately prior to transfusion to a recipient. Addition of oxygen immediately prior to transfusion is beneficial to recipient of RBCs. Oxygen addition is particularly beneficial to recipients of massive transfusions such as those who suffer from sickle cell disease.

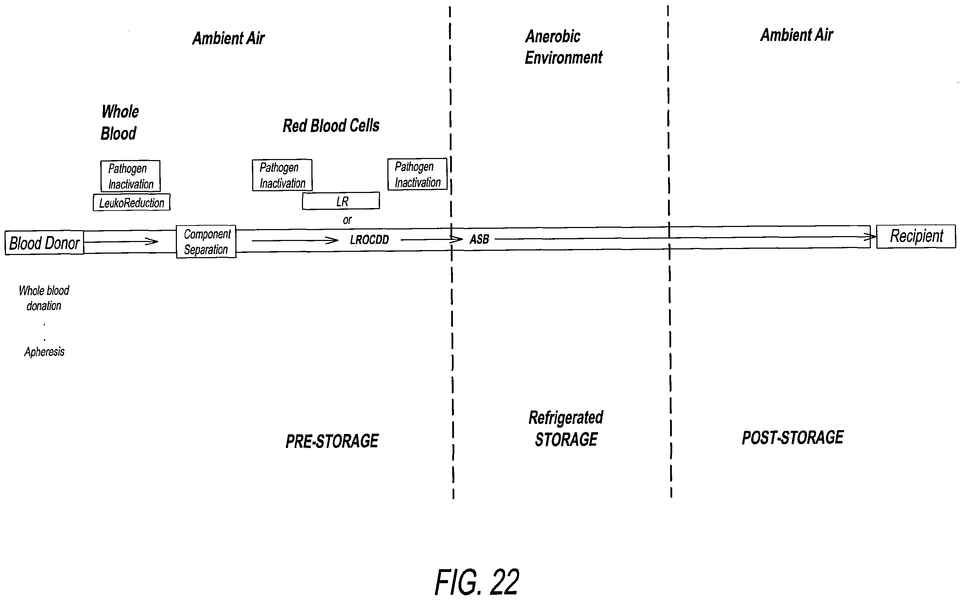

FIG. 22 illustrates a configuration of the flowchart of FIG. 1, including leukoreduction, oxygen, carbon dioxide or oxygen and/or carbon dioxide depletion, and pathogen inactivation at various possible times during collection and storage. Pathogen inactivation may harm RBCs by generating reactive oxygen species during the process. Anaerobic environments reduce ROS RBC damage during subsequent storage period.

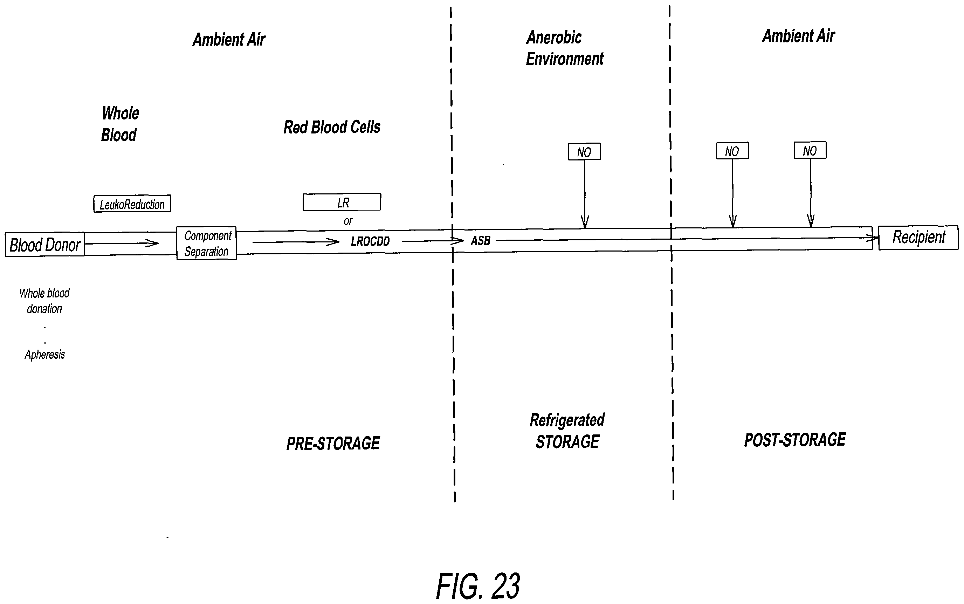

FIG. 23 illustrates a configuration of flowchart of FIG. 1, including leukoreduction, oxygen, carbon dioxide or oxygen and/or carbon dioxide depletion, and nitric oxide addition at various possible times during storage. Nitric oxide can be added as NO-precursors, NO gas or nitrate to anaerobic RBCs. The nitric oxide and hemoglobin-NO compound are less labile under anaerobic conditions.

## Claims

1. A method for preparing red blood cells (RBCs) comprising:
separating RBCs from whole blood to form packed RBCs;
reducing oxygen and carbon dioxide from said packed RBCs to form oxygen and carbon dioxide-reduced packed RBCs;
inactivating pathogens in said packed RBCs with riboflavin and light therapy after said removing oxygen and carbon dioxide;
storing said oxygen and carbon dioxide-reduced packed RBCs in an anaerobic storage environment; and
irradiating said packed RBCs with gamma or x-ray irradiation, wherein said irradiation occurs before, during, or after said storing;
wherein said reducing oxygen and carbon dioxide from said packed RBCs comprises passing said packed RBCs through an oxygen and carbon dioxide depletion device,
wherein said irradiation before storing occurs after said reducing oxygen and carbon dioxide.

2. The method of claim 1, further comprising adding an additive solution to said packed RBCs prior to said reducing oxygen and carbon dioxide from said packed RBCs to form a suspension of RBCs.

3. The method of claim 2, wherein said additive solution has a pH from 5.0 to 9.0.

4. The method of claim 2, wherein said additive solution further comprises an antioxidant selected from the group consisting of quercetin, alpha-tocopheral, ascorbic acid, and enzyme inhibitor for oxidase.

5. A method of any one of the preceding claims, further comprising one or more steps selected from the group consisting of:
adding nutrients or metabolic supplements to said oxygen and carbon dioxide-reduced packed RBCs, or oxygen and carbon dioxide-reduced suspension of RBCs during storage in said anaerobic storage environment, and
reoxygenating the stored oxygen and carbon dioxide-reduced packed RBCs, or oxygen and carbon dioxide-reduced suspension of RBCs to form oxygenated packed RBCs or an oxygenated suspension of RBCs for transfusion.

6. A method of any one of the preceding claims, wherein said oxygen and carbon dioxide depletion device comprises:
a housing,
a plurality of structures selected from the group consisting of hollow fibers, gas-permeable films, and gas permeable fibers, and any combinations thereof extending within the housing from an entrance to an exit thereof; wherein said plurality of structures are formed of a material that is permeable to both oxygen and carbon dioxide and are adapted to receiving and conveying RBCs; and
an amount of an oxygen sorbent and carbon dioxide sorbent packed within the housing and contiguous to said plurality of structures.

7. A method of any one of the preceding claims, wherein said anaerobic storage environment further comprises a carbon dioxide depleted storage environment containing oxygen sorbents and carbon dioxide sorbents for maintaining said oxygen and carbon dioxide-reduced packed RBCs or said oxygen and carbon dioxide-reduced suspension of RBCs, in an oxygen and carbon dioxide depleted storage environment.

8. A method of any one of the preceding claims, wherein said anaerobic storage environment comprises an oxygen-impermeable storage bag containing an oxygen and carbon dioxide sorbent to maintain said oxygen and carbon dioxide-reduced packed RBCs, or said oxygen and carbon dioxide- reduced suspension of RBCs, in an oxygen and carbon dioxide reduced condition.

9. A method of any one of the preceding claims, further comprising leukoreducing said whole blood, said packed RBCs, or said suspension of RBCs,
wherein said leukoreduction occurs prior to or after reducing oxygen and carbon dioxide, and before, during or after said storing.

10. The method of claim 9, further comprising one or more steps selected from the group consisting of:
editing a composition selected from the group consisting of said whole blood before said storing, said packed RBCs before said storing, said suspension of RBCs before said storing, the stored oxygen and carbon dioxide-reduced packed RBCs, and the stored oxygen and carbon dioxide- reduced suspension of RBCs,
adding nitric oxide or NO precursor molecules to said oxygen and carbon dioxide reduced packed RBCs, or oxygen and carbon dioxide- reduced suspension of RBCs during or after said storing, and
reducing the volume of said oxygen and carbon-dioxide reduced packed RBCs, or oxygen and carbon dioxide- reduced suspension of RBCs after said storing.

11. The method of claim 10, wherein said editing comprises identifying and removing moribund RBCs,
wherein said identifying moribund RBCs is selected from the group consisting of:
removing the densest 10% of RBCs by filtration;
osmotically shocking RBCs and removing damaged RBCs;
removing RBCs by high affinity ligand filtration; and
separating RBC with reduced deformability by processing through a bump array microfluidic device;
wherein said editing occurs before said separating RBCs from whole blood, after said separating RBCs from whole blood, before said storing, or after said storing.

12. The method of claim 11, wherein said high affinity ligand filtration comprises binding of said RBC using a high affinity ligand that recognizes clustered Band-3 or phosphatidylserine on the surface of said RBC.

13. The method of claim 1, further comprising:
editing a composition selected from the group consisting of said whole blood before said storing, said packed RBCs before said storing, and the stored oxygen and carbon dioxide-reduced RBCs;
reducing the volume of said stored oxygen and carbon dioxide-reduced packed RBCs prior to reoxygenation; and
adding nitric oxide to said stored oxygen and carbon dioxide-reduced packed RBCs when said oxygen and carbon dioxide-reduced packed RBCs are stored in said anaerobic storage environment.

14. The method of claim 1, wherein said irradiating said packed RBCs with gamma or x-ray irradiation occurs after said reducing oxygen and carbon dioxide and before said storing.

15. The method of claim 1, wherein said irradiating said packed RBCs with gamma or x-ray irradiation occurs during said storing.

## Patentansprüche

1. Verfahren zur Herstellung von Erythrozyten (Erys), umfassend:
Trennen von Erys von Vollblut zur Bildung von Ery-Konzentraten;
Reduzieren von Sauerstoff und Kohlendioxid aus den Ery-Konzentraten zur Bildung von Sauerstoff- und Kohlendioxid-reduzierten Ery-Konzentraten;
Inaktivieren von Erregern in den Ery-Konzentraten mit Riboflavin und Lichttherapie nach der Entfernung von Sauerstoff und Kohlendioxid;
Lagern der Sauerstoff- und Kohlendioxid-reduzierten Ery-Konzentrate in einer anaeroben Lagerungsumgebung; und
Bestrahlen der Ery-Konzentrate mit Gamma- oder Röntgenstrahlung, wobei die Bestrahlung vor, während oder nach der Lagerung erfolgt;
wobei das Reduzieren von Sauerstoff und Kohlendioxid aus den Ery-Konzentraten das Durchleiten der Ery-Konzentrate durch eine Sauerstoff- und Kohlendioxid-Abreicherungsvorrichtung umfasst, wobei die Bestrahlung vor der Lagerung nach dem Reduzieren von Sauerstoff und Kohlendioxid erfolgt.

2. Verfahren nach Anspruch 1, ferner umfassend das Hinzufügen einer Additivlösung zu den Ery-Konzentraten vor dem Reduzieren von Sauerstoff und Kohlendioxid aus den Ery-Konzentraten zur Bildung einer Suspension von Erys.

3. Verfahren nach Anspruch 2, wobei die Additivlösung einen pH von 5,0 bis 9,0 aufweist.

4. Verfahren nach Anspruch 2, wobei die Additivlösung ferner ein Antioxidans umfasst, das aus der aus Quercetin, alpha-Tocopheral, Ascorbinsäure und Enzyminhibitor für Oxidase bestehenden Gruppe ausgewählt ist.

5. Verfahren nach einem der vorhergehenden Ansprüche, ferner umfassend einen oder mehrere Schritte, ausgewählt aus der folgenden Gruppe:
Hinzufügen von Nährstoffen oder metabolischen Ergänzungsmitteln zu den Sauerstoff- und Kohlendioxid-reduzierten Ery-Konzentraten, oder zur Sauerstoff- und Kohlendioxid-reduzierten Suspension von Erys während der Lagerung in der anaeroben Lagerungsumgebung, und
Reoxygenieren der gelagerten Sauerstoff- und Kohlendioxid-reduzierten Ery-Konzentrate, oder der Sauerstoff- und Kohlendioxid-reduzierten Suspension von Erys zur Bildung von oxygenierten Ery-Konzentraten oder einer oxygenierten Suspension von Erys für eine Transfusion.

6. Verfahren nach einem der vorhergehenden Ansprüche, wobei die Sauerstoff- und Kohlendioxid-Abreicherungsvorrichtung umfasst:
ein Gehäuse,
eine Vielzahl von Strukturen, ausgewählt aus der Gruppe von Hohlfasern, gasdurchlässigen Folien und gasdurchlässigen Fasern, sowie beliebigen Kombinationen davon, die sich innerhalb des Gehäuses von einem Eingang zu einem Ausgang davon erstrecken; wobei die Vielzahl von Strukturen aus einem Material besteht, das sowohl für Sauerstoff als auch für Kohlendioxid durchlässig ist, und zum Aufnehmen und Transportieren von Erys ausgelegt ist; und
eine Menge eines Sauerstoff-Sorbens und eines Kohlendioxid-Sorbens, die im Gehäuse gepackt sind und an die Vielzahl von Strukturen angrenzen.

7. Verfahren nach einem der vorhergehenden Ansprüche, wobei die anaerobe Lagerungsumgebung ferner eine Kohlendioxid-abgereicherte Lagerungsumgebung umfasst, die Sauerstoff-Sorbentien und Kohlendioxid-Sorbentien zur Aufrechterhaltung der Sauerstoff- und Kohlendioxid-reduzierten Ery-Konzentrate oder der Sauerstoff- und Kohlendioxid-reduzierten Suspension von Erys in einer Sauerstoff- und Kohlendioxidabgereicherten Lagerungsumgebung enthält.

8. Verfahren nach einem der vorhergehenden Ansprüche, wobei die anaerobe Lagerungsumgebung einen sauerstoffundurchlässigen Aufbewahrungsbeutel umfasst, der ein Sauerstoff- und Kohlendioxid-Sorbens zur Aufrechterhaltung der Sauerstoff- und Kohlendioxid-reduzierten Ery-Konzentrate oder der Sauerstoff- und Kohlendioxid-reduzierten Suspension von Erys in einem Sauerstoff- und Kohlendioxid-reduzierten Zustand enthält.

9. Verfahren nach einem der vorhergehenden Ansprüche, ferner umfassend Leukoreduzieren des Vollbluts, der Ery-Konzentrate oder der Suspension von Erys,
wobei die Leukoreduktion vor oder nach dem Reduzieren von Sauerstoff und Kohlendioxid,
und vor, während oder nach der Lagerung erfolgt.

10. Verfahren nach Anspruch 9, ferner umfassend einen oder mehrere Schritte, ausgewählt aus der folgenden Gruppe:
Bearbeiten einer Zusammensetzung, ausgewählt aus der Gruppe, die besteht aus dem Vollblut vor der Lagerung, den Ery-Konzentraten vor der Lagerung, der Suspension von Erys vor der Lagerung, den gelagerten Sauerstoff- und Kohlendioxid-reduzierten Ery-Konzentraten und der gelagerten Sauerstoff- und Kohlendioxid-reduzierten Suspension von Erys,
Hinzufügen von Stickoxid- oder NO-Vorläufermolekülen zu den Sauerstoff- und Kohlendioxid-reduzierten Ery-Konzentraten, oder der Sauerstoff- und Kohlendioxid-reduzierten Suspension von Erys während oder nach der Lagerung, und
Reduzieren des Volumens der Sauerstoff- und Kohlendioxid-reduzierten Ery-Konzentrate, oder der Sauerstoff- und Kohlendioxid-reduzierten Suspension von Erys nach der Lagerung.

11. Verfahren nach Anspruch 10, wobei die Bearbeitung die Identifizierung und Entfernung von moribunden Erys umfasst,
wobei die Identifizierung moribunder Erys aus der folgenden Gruppe ausgewählt ist:
Entfernen der dichtesten 10% von Erys durch Filtration;
osmotisches Schocken von Erys und Entfernen von geschädigten Erys;
Entfernen von Erys durch Hochaffinitäts-Liganden-Filtration; und
Abtrennen von Erys mit reduzierter Verformbarkeit durch Bearbeitung durch eine Bump-Array-Mikrofluidikvorrichtung;
wobei die Bearbeitung vor dem Trennen von Erys von Vollblut, nach dem Trennen von Erys von Vollblut, vor der Lagerung oder nach der Lagerung erfolgt.

12. Verfahren nach Anspruch 1, wobei die Hochaffinitäts-Liganden-Filtration die Bindung der Erys unter Verwendung eines hochaffinen Liganden, der Band-3-Cluster oder Phosphatidylserin auf der Oberfläche der Erys erkennt, umfasst.

13. Verfahren nach Anspruch 1, ferner umfassend:
Bearbeiten einer Zusammensetzung, die ausgewählt ist aus der Gruppe von Vollblut vor der Lagerung, den Ery-Konzentraten vor der Lagerung und den gelagerten Sauerstoff- und Kohlendioxid-reduzierten Erys;
Reduzieren des Volumens der gelagerten Sauerstoff- und Kohlendioxid-reduzierten Ery-Konzentrate vor der Reoxygenierung; und
Hinzufügen von Stickoxid zu den gelagerten Sauerstoff- und Kohlendioxid-reduzierten Ery-Konzentraten, wenn die Sauerstoff- und Kohlendioxid-reduzierten Ery-Konzentrate in der anaeroben Lagerungsumgebung gelagert werden.

14. Verfahren nach Anspruch 1, wobei die Bestrahlung der Ery-Konzentrate mit Gamma- oder Röntgenstrahlung nach der Reduzierung von Sauerstoff und Kohlendioxid und vor der Lagerung erfolgt.

15. Verfahren nach Anspruch 1, wobei die Bestrahlung der Ery-Konzentrate mit Gamma- oder Röntgenstrahlung während der Lagerung erfolgt.

## Revendications

1. Procédé pour la préparation de globules rouges (RBC) comprenant :
la séparation de RBC de sang entier pour former des RBC concentrés ;
l'appauvrissement en oxygène et en dioxyde de carbone desdits RBC concentrés pour former des RBC concentrés appauvris en oxygène et en dioxyde de carbone ;
l'inactivation de pathogènes dans lesdits RBC concentrés avec de la riboflavine et une thérapie légère après ladite élimination d'oxygène et de dioxyde de carbone ;
le stockage desdits RBC concentrés appauvris en oxygène et en dioxyde de carbone dans un environnement de stockage anaérobe ; et
l'irradiation desdits RBC concentrés avec une irradiation gamma ou rayons X, ladite irradiation ayant lieu avant, pendant ou après ledit stockage ;
ledit appauvrissement en oxygène et en dioxyde de carbone desdits RBC concentrés comprenant le passage desdits RBC concentrés à travers un dispositif d'appauvrissement en oxygène et en dioxyde de carbone,
ladite irradiation avant le stockage ayant lieu après ledit appauvrissement en oxygène et en dioxyde de carbone.

2. Procédé selon la revendication 1, comprenant en outre l'ajout d'une solution d'additif auxdits RBC concentrés avant ledit appauvrissement en oxygène et en dioxyde de carbone desdits RBC concentrés pour former une suspension de RBC.

3. Procédé selon la revendication 2, ladite solution d'additif possédant un pH de 5,0 à 9,0.

4. Procédé selon la revendication 2, ladite solution d'additif comprenant en outre un antioxydant choisi dans le groupe constitué par la quercétine, l'alpha-tocophérol, l'acide ascorbique et un inhibiteur enzymatique pour une oxydase.

5. Procédé selon l'une quelconque des revendications précédentes, comprenant en outre une ou plusieurs étapes choisies dans le groupe constitué par :
l'ajout de nutriments ou de suppléments métaboliques auxdits RBC concentrés appauvris en oxygène et en dioxyde de carbone, ou à ladite suspension de RBC appauvris en oxygène et en dioxyde de carbone pendant le stockage dans ledit environnement de stockage anaérobe, et
la réoxygénation des RBC concentrés appauvris en oxygène et en dioxyde de carbone stockés, ou de la suspension de RBC appauvris en oxygène et en dioxyde de carbone pour former des RBC concentrés oxygénés ou une suspension de RBC oxygénés pour une transfusion.

6. Procédé selon l'une quelconque des revendications précédentes, ledit dispositif d'appauvrissement en oxygène et en dioxyde de carbone comprenant :
un boîtier,
une pluralité de structures choisies dans le groupe constitué par des fibres creuses, des films perméables aux gaz et des fibres perméables aux gaz, et de quelconques combinaisons correspondantes s'étendant à l'intérieur du boîtier depuis l'entrée jusqu'à une sortie de celui-ci ; ladite pluralité de structures étant formées d'un matériau qui est perméable à la fois à l'oxygène et au dioxyde de carbone et étant adaptées pour recevoir et acheminer des RBC ; et
une quantité d'un sorbant d'oxygène et d'un sorbant de dioxyde de carbone compactée à l'intérieur du boîtier et contigüe à ladite pluralité de structures.

7. Procédé selon l'une quelconque des revendications précédentes, ledit environnement de stockage anaérobe comprenant en outre un environnement de stockage appauvri en dioxyde de carbone contenant des sorbants d'oxygène et des sorbants de dioxyde de carbone pour maintenir les RBC concentrés appauvris en oxygène et en dioxyde de carbone ou la suspension de RBC appauvris en oxygène et en dioxyde de carbone dans un environnement de stockage appauvri en oxygène et en dioxyde de carbone.

8. Procédé selon l'une quelconque des revendications précédentes, ledit environnement de stockage anaérobe comprenant un sac de stockage imperméable à l'oxygène contenant un sorbant d'oxygène et de dioxyde de carbone pour maintenir lesdits RBC concentrés appauvris en oxygène et en dioxyde de carbone ou la suspension de RBC appauvris en oxygène et en dioxyde de carbone dans une condition appauvrie en oxygène et en dioxyde de carbone.

9. Procédé selon l'une quelconque des revendications précédentes, comprenant en outre une leucoréduction dudit sang entier, desdits RBC concentrés ou de ladite suspension de RBC,
ladite leucoréduction ayant lieu avant ou après l'appauvrissement en oxygène et en dioxyde de carbone, et avant, pendant ou après ledit stockage.

10. Procédé selon la revendication 9, comprenant en outre une ou plusieurs étapes choisies dans le groupe constitué par :
la modification d'une composition choisie dans le groupe constitué par ledit sang entier avant ledit stockage, lesdits RBC concentrés avant ledit stockage, ladite suspension de RBC avant ledit stockage, les RBC concentrés appauvris en oxygène et en dioxyde de carbone stockés, et la suspension de RBC appauvris en oxygène et en dioxyde de carbone stockée,
l'ajout d'oxyde nitrique ou de molécules précurseurs de NO auxdits RBC concentrés appauvris en oxygène et en dioxyde de carbone, ou à ladite suspension de RBC appauvris en oxygène et en dioxyde de carbone pendant ou après ledit stockage, et
la réduction du volume desdits RBC concentrés appauvris en oxygène et en dioxyde de carbone, ou de ladite suspension de RBC appauvris en oxygène et en dioxyde de carbone après ledit stockage.

11. Procédé selon la revendication 10, ladite modification comprenant l'identification et l'élimination de RBC moribonds,
ladite identification de RBC moribonds étant choisie dans le groupe constitué par :
l'élimination des 10 % les plus denses des RBC par filtration ;
le choc de manière osmotique des RBC et l'élimination des RBC endommagés ;
l'élimination de RBC par filtration à ligand de haute affinité ; et
la séparation de RBC comportant une déformabilité réduite par traitement à travers un dispositif microfluidique à réseau de protubérances ;
ladite modification ayant lieu avant ladite séparation des RBC du sang entier, après ladite séparation des RBC du sang entier, avant ledit stockage, ou après ledit stockage.

12. Procédé selon la revendication 11, ladite filtration à ligand de haute affinité comprenant la liaison dudit RBC en utilisant un ligand de haute affinité qui reconnaît Band-3 mis en cluster ou la phosphatidylsérine sur la surface dudit RBC.

13. Procédé selon la revendication 1, comprenant en outre :
la modification d'une composition choisie dans le groupe constitué par ledit sang entier avant ledit stockage, lesdits RBC concentrés avant ledit stockage, et les RBC appauvris en oxygène et en dioxyde de carbone stockés ;
la réduction du volume desdits RBC concentrés appauvris en oxygène et en dioxyde de carbone stockés avant la réoxygénation ; et
l'ajout d'oxyde nitrique auxdits RBC concentrés appauvris en oxygène et en dioxyde de carbone stockés lorsque lesdits RBC concentrés appauvris en oxygène et en dioxyde de carbone sont stockés dans ledit environnement de stockage anaérobe.

14. Procédé selon la revendication 1, ladite irradiations desdits RBC concentrés avec une irradiation gamma ou rayons X ayant lieu après ledit appauvrissement en oxygène et en dioxyde de carbone et avant ledit stockage.

15. Procédé selon la revendication 1, ladite irradiations desdits RBC concentrés avec une irradiation gamma ou rayons X ayant lieu pendant ledit stockage.
